# EUROPEAN PATENT SPECIFICATION

(11) **EP 4 308 904 B1**
(45) Date of publication and mention of the grant of the patent: **11.11.2026**
(21) Application number: 22717961.1
(22) Date of filing: 16.03.2022
(51) Int. Cl.: G01N 21/31, G01N 33/02, G06Q 10/06, G06Q 10/08, G06Q 30/06, G06Q 50/02, G06V 10/58, G06V 10/82, G06V 20/68, G01N 21/84, A01D 91/04

(54) **METHOD FOR GENERATING A TRAINED MODEL TO DETERMINE A RIPENESS METRIC OF A FOOD ITEM AND SYSTEM FOR DETERMINING RIPENESS LEVELS FOR FOOD ITEMS USING NON-CONTACT ASSESSMENTS OF THE FOOD ITEMS**
VERFAHREN ZUR ERSTELLUNG EINES TRAINIERTEN MODELLS ZUR BESTIMMUNG EINES REIFEGRADS FÜR EIN LEBENSMITTEL UND SYSTEM ZUR BESTIMMUNG DES REIFEGRADS VON LEBENSMITTELN UNTER VERWENDUNG BERÜHRUNGSLOSER MESSUNGEN DER LEBENSMITTEL
PROCÉDÉ DE GÉNÉRATION D'UN MODÈLE APPRIS POUR DÉTERMINER UN INDICE DE MATURITÉ D'UN PRODUIT ALIMENTAIRE ET SYSTÈME DE DÉTERMINATION DES NIVEAUX DE MATURITÉ DE PRODUITS ALIMENTAIRES À L'AIDE D'ÉVALUATIONS SANS CONTACT DE CES PRODUITS

(30) Priority: 16.03.2021 US 202163161507 P
(43) Date of publication of application: 24.01.2024
(73) Proprietor: Apeel Technology, Inc., Goleta, California 93117 (US)
(72) Inventor: PATTISON, Richard, Goleta, California 93117 (US); KAHLSCHEUER, Matthew, Goleta, California 93117 (US)
(74) Representative: dompatent
(86) International application number: PCT/US2022/020522
(87) International publication number: WO 2022/197785

(56) References cited:
- US-A1- 2018 365 820
- US-A1- 2019 340 749
- GANAPATHY DR KIRUPA ET AL: "Smart Marketing Systems for Fruits using Wireless Sensors", 2018 IEEE INTERNATIONAL CONFERENCE ON SYSTEM, COMPUTATION, AUTOMATION AND NETWORKING (ICSCA), IEEE, 6 July 2018 (2018-07-06), pages 1 - 4, XP033450774, DOI: 10.1109/ICSCAN.2018.8541217

## Description

### RELATED APPLICATIONS

This application claims priority to U.S. Provisional Application Serial No. 63/161,507, filed on March 16, 2021.

### TECHNICAL FIELD

This document describes methods and systems related to determining a food item ripeness metric from a combination of measurements that includes destructive or invasive measurements and building a model to non-destructively predict the ripeness of a food item.

### BACKGROUND

Firmness of a food item, such as fruit and produce, can be a useful indication of suitability of the food item for consumption. Firmness of the food item can correlate to ripeness. Whether the food item is ripe can indicate whether it is ready to be consumed. When food items are ripe, they can be sold to consumers at grocery stores and similar establishments. Sometimes, price of the food items can also change depending on the ripeness of the food items.

Sometimes, when food items are overripe, these food items may not be sold to customers in stores such as grocery stores and farmers markets. Instead, the food items may be delivered to food processing plants to be used in processed foods. The food items can also be sold to consumers but at a lower price. When food items are not yet ripe, the food items may be stored for longer periods of time or actively ripened (e.g., by ethylene exposure) before such food items can be sold or otherwise delivered to consumers.

Consumers can test ripeness of food items while they are shopping. For example, a consumer can press or squeeze on the food item to determine how soft or hard the food item is.
Other individuals in a supply chain can similarly press or squeeze on the food item to determine its firmness. Grocers can squeeze food items to determine whether the food items may be ready to put on the shelf for purchase by consumers. Warehouse workers can also squeeze food items to determine whether the foot items should be moved for outbound shipment to consumers or food processing plants. Pressing or squeezing on the food items may not always be an accurate indication of how firm or ripe the food items are.

Moreover, pressing or squeezing on the food items can be a destructive way to test the firmness or ripeness of the food items. Sometimes, firmness or ripeness of the food items can also be tested using specialized devices. Such devices can measure firmness by puncturing skin of the food items. The devices can also measure firmness by removing portions of the skin of the food items. These devices can provide destructive techniques to test firmness and/or ripeness of the food items. Destructive techniques can result in a tradeoff between measurement reliability and resulting losses from large required sample sizes. Destructive techniques can also be time consuming with limited options for automated data collection. Destructive techniques may be user-dependent measurements that may not be automated or integrated with other quality tools. Food item firmness can also be tracked throughout their ripening period for holdbacks set at different customer sites as a way to measure food item quality and senescence behavior. This can be a time-consuming and capital-intensive way to measure food item quality.

US 2019/340749 A1 relates to using a computing device and sensors to determine attributes and characteristics of a vegetables or fruits.

### SUMMARY

The present invention is directed to a method for generating a trained model to determine a ripeness metric of a food item as defined in appended independent claim 1. The present invention is also directed to a system for determining ripeness levels for food items using non-contact assessments of the food items, the system comprising, amongst other features defined in appended independent claim 15, at least one computing system configured to perform the method of claim 1. Particular embodiments of the invention are defined in the appended dependent claims. The scope of the present invention is solely restricted and defined by the appended claims.

This document generally describes systems, methods, and techniques for predicting ripeness of a food item non-destructively (e.g., using a spectrometer). In particular, the disclosed technology can use non-destructive metrology in tandem with machine learning models trained on data to more accurately determine food item ripeness without having to puncture or otherwise destroy the food item. A ripeness metric can be engineered using data acquired from multiple different invasive, destructive tools, such as penetrometers and durometers. Thus, multiple different measurements can be correlated during food item ripening. This ripeness metric can become a desired output of the techniques described herein. Machine learning models can therefore be trained to identify the ripeness metric using non-destructive data as inputs. Data can be collected to train the models to take non-destructive data, such as spectra, as an input to then predict the ripeness metric for a food item in real-time. The models can be trained to map spectra data to the engineered ripeness metric. Once the models are trained, the models can be applied in real-time to predict ripeness of a food item using non-destructive, spectra data of the food item.

Using the models can be advantageous to provide non-destructive, fast, and reliable determination of food items' ripeness (e.g., firmness, consumption suitability), which can be important for suppliers and retailers to determine, for example, how to move these items through a supply chain. For example, such ripeness determinations can factor into supply chain decisions regarding which food items to select for distribution to different food item sellers and consumers, such as selecting and distributing a first group of high quality food items (e.g., desirable ripeness and taste qualities) for direct consumer purchase, and a second group of lower quality food items (e.g., less desirable ripeness and taste quality) for other uses, such as industrial food processing and manufacturing.

The disclosed technology can provide for measuring food item ripeness using a variety of input data. For example, a ripeness metric can be engineered using destructive or invasive measurements. The destructive or invasive measurements can be received from a penetrometer, a durometer, and/or the like. One or more machine learning models can then be generated to predict the engineered ripeness metric using non-destructive measurements. The non-destructive measurements can include spectrometer data and/or historical information about the food item. In real-time, spectrometer data about a food item or a batch of food items can be collected and used as input for the one or more models to predict the ripeness of the food item or batch of food items.

Spectroscopic techniques, such as Visible and Near-Infrared (NIR) spectrometry, can be advantageous to measure food item characteristics. Spectroscopic techniques as described herein can use a light source capable of penetrating into flesh of a specimen (e.g., fruit, produce, other food items), and a detector capable of measuring absorbance of a highly and precisely discretized set of spectral bands. As chemical structures change within the specimen (e.g., due to breakdown of cellulosic material into monomeric, soluble sugars), there can be minute changes in spectral absorbance profiles. This information can be collected and used with the models to non-destructively measure ripeness of the corresponding specimen. Moreover, multivariate regression techniques can be used to detect differences between absorbance profiles. Spectral measurements can therefore be used to determine NIR-based firmness/ripeness levels of different types of food items.

Different quality metrics can be determined using NIR-based firmness/ripeness level techniques described herein. For example, avocado dry matter can be an indicator of fruit maturity and used by growers to determine when their trees are ready to harvest. Additionally, apple firmness can be a quality metric utilized to determine shelf-life extension for apples. The disclosed technology can be used to non-destructively, accurately, and quickly determine quality metrics associated with not just avocados and apples, but also other types of food items, fruits, and produce.

Moreover, the disclosed technology can be used with hyperspectral imaging techniques to improve ripening predictions. Hyperspectral cameras can extend capability of collecting NIR spectroscopic measurements over spatial domains, as opposed to a single point. This spatial resolution of spectral profiles can allow for many pixels of spectra to be collected for a single piece of food item. Additionally, hyperspectral imaging cameras can be implemented inline such that the cameras collect data on many pieces of food items that can be moving simultaneously along a conveyor belt. Using the techniques described herein, food items can be categorized based on their ripeness. For example, the model can be trained to distinguish food items that ripen quickly from food items that ripen more slowly.

Preferred embodiments described herein can include systems and methods for determining ripeness levels for food items using non-contact assessments of the food items. The method can include receiving, by a computing system and from a spectral imaging device, spectral data of a food item, filtering, by the computing system, the spectral data, determining, by the computing system and based on applying a trained model to the filtered spectral data, a ripeness level of the food item. The trained model can be trained using (i) one or more destructive measurements of other food items and (ii) spectral data for the other food items. The other food items can be of a same food type as the food item. The ripeness level of the food item can be determined without taking destructive measurements of the food item. The method can also include transmitting, to a user computing device, the ripeness level of the food item for display at the user computing device.

The preferred embodiments can include one or more following features. The spectral data can include one or more non-destructive measurements of the food item. The trained model can include one or more layers. Each of the layers can include (i) training images of the other food items and (ii) labels that indicate food item classifications for each of the other food items depicted by the training images. Filtering the spectral data can include trimming the spectral data, scaling the spectral data, and applying a Savitzky-Golay 2^{nd} derivative filter to the spectral data to reduce noise.

Furthermore, the method can include determining that the food item is suitable for consumption based on the ripeness level of the food item exceeding a threshold value. The method can also include determining that the food item is not suitable for consumption based on the ripeness level of the food item being less than a threshold value. The food item can be at least one of an avocado, an apple, and a berry.

In some implementations, the spectral imaging device can be a point spectrometer capable of capturing spectral data using light having a wavelength of 534 nm to 942 nm. In some implementations, the spectral imaging device can be a point spectrometer capable of capturing spectral data using light having a wavelength of 530 nm to 950 nm. The point spectrometer can also capture the spectral data using light having a wavelength of 690 nm to 912 nm. The point spectrometer can capture the spectral data using light having a wavelength of 672 nm to 948 nm.

The ripeness level of the food item can further based on input data that includes a place of origin of the food item, a storage temperature of the food item, and historic ripening information associated with the food item.

The present invention, as defined in appended claims 1 and 15, is directed to systems and methods for generating a trained model to determine a ripeness metric of a food item. The method includes receiving, by a computing system, (i) penetrometer data from one or more penetrometers and (ii) durometer data from one or more durometers for a plurality of test food items of a same food type, selecting portions of the penetrometer data and the durometer data, determining a ripeness metric for food items of the same food type based on the selected portions of the penetrometer data and the durometer data, and generating a machine learning trained model based on the ripeness metric. The machine learning trained model can correlate destructive measurements provided by the selected portions of the penetrometer data and the selected portions of the durometer data with non-destructive measurements provided by spectral data to model the ripeness metric for the food items of the same food type.

The preferred embodiments can include one or more of the following features. For example, selecting portions of the penetrometer data and the durometer data can include plotting the penetrometer data and the durometer data, identifying an inflection point in the plotted penetrometer data and the plotted durometer data, selecting portions of the penetrometer data and the durometer data based on the inflection point, and discarding unselected portions of the penetrometer data and the durometer data. Selecting portions of the penetrometer and durometer data based on the inflection point can include selecting portions of the penetrometer data before the inflection point and selecting portions of the durometer data after the inflection point. Discarding portions of the durometer data before the inflection point and discarding portions of the penetrometer data after the inflection point.

Moreover, generating the machine learning trained model can include (i) correlating the selected portions of the penetrometer data before the inflection point with one or more wavelengths of spectral data that correspond to the plurality of test food items that are hard and (ii) correlating the selected portions of the durometer data after the inflection point with one or more wavelengths of spectral data that correspond to the test food items that are soft.

As another example, the machine learning trained model further can correlate destructive measurements provided by the selected portions of the penetrometer data and the selected portions of the durometer data with at least one of (i) a place of origin, (ii) a storage temperature, and (iii) historic ripening information associated with the food items of the same food type.

Preferred embodiments described herein can also include systems and methods for modifying a supply chain based on ripeness levels of food items. The method can include receiving, by a computing system, a ripeness level of a food item of a food item type. The ripeness level can be determined using a non-destructive measurement of the food item and a trained model for the food item type. The trained model can be trained using one or more destructive measurements of other food items of the food item type. The method can also include identifying supply chain information for the food item that includes a preexisting supply chain schedule and destination for the food item, determining whether to modify the supply chain information for the food item based on the received ripeness level, and in response to a determination to modify the supply chain information, generating modified supply chain information based on the received ripeness level. The modified supply chain information can include one or more of a modified supply chain schedule and modified destination for the food item. The method can also include transmitting the modified supply chain information to one or more supply chain actors to implement the modified supply chain information.

The preferred embodiments can include one or more of the following features. The method can further include determining, based on the received ripeness level exceeding a threshold value, that the food item is suitable for consumption by end-consumers, and determining, based on the received ripeness level being less than the threshold value, that the food item is not suitable for consumption by the end-consumers.

As another example, the modified supply chain information can include instructions that, when executed by the one or more supply chain actors, cause the food item to be moved for outbound shipment to end-consumers that are geographically closest to a location of the food item. The modified supply chain information can also include instructions that, when executed by the one or more supply chain actors, cause the food item to be moved for outbound shipment to a food processing plant.

The present invention, as defined in appended claims 1 and 15, is directed to systems and methods for determining ripeness levels for food items using non-contact assessments of the food items. The system includes one or more penetrometers that can measure penetrometer data for a plurality of test food items of a same food type, one or more durometers that can measure durometer data for the plurality of test food items of the same food type, one or more spectral imaging devices that can measure spectral data for food items of the same food type, and at least one computing system configured to perform the method of any one of claims 1 to 12. The computing system can receive the penetrometer data and the durometer data, select portions of the penetrometer data and the durometer data, determine a ripeness metric for the food items of the same food type based on the selected portions of the penetrometer data and the durometer data, and generate a machine learning trained model based on the ripeness metric. The machine learning trained model can correlate destructive measurements provided by the selected portions of the penetrometer data and the selected portions of the durometer data with non-destructive measurements provided by spectral data to model the ripeness metric for the food items of the same food type. The computing system can also receive, from the one or more spectral imaging devices, spectral data of a food item of the same food type, filter the spectral data of the food item of the same food type, and determine, based on applying the machine learning trained model to the filtered spectral data of the food item of the same food type, a ripeness level of the food item. The ripeness level of the food item can be determined without taking destructive measurements of the food item. Moreover, the computing system can identify supply chain information for the food item that includes a preexisting supply chain schedule and destination for the food item, determine whether to modify the supply chain information for the food item based on the ripeness level of the food item, and in response to a determination to modify the supply chain information, generate modified supply chain information based on the ripeness level of the food item. The modified supply chain information can include one or more of a modified supply chain schedule and modified destination for the food item. The computing system can also transmit, to a user computing device, (i) the ripeness level of the food item and (ii) the modified supply chain information for display at the user computing device.

The preferred embodiments described herein can include one or more of the following features. For example, the one or more spectral imaging devices can include a point spectrometer. The machine learning trained model can include one or more layers. Each of the layers can include (i) training images of the plurality of test food items of the same food type and (ii) labels that indicate food item classifications for each of the plurality of test food items depicted by the training images.

As another example, the computing system can also determine that the food item is suitable for consumption based on the ripeness level of the food item exceeding a threshold value. The computing system can determine that the food item is not suitable for consumption based on the ripeness level of the food item being less than a threshold value. The computing system can further generate the machine learning trained model based on (i) correlating the selected portions of the penetrometer data with one or more wavelengths of spectral data that correspond to plurality of test food items that are hard and (ii) correlating the selected portions of the durometer data with one or more wavelengths of spectral data that correspond to the plurality of test food items that are soft.

As yet another example, the modified supply chain information can include instructions that, when executed by one or more supply chain actors at the user computing device, cause the food item to be moved for outbound shipment to end-consumers that are geographically closest to a location of the food item. The modified supply chain information can also include instructions that, when executed by the one or more supply chain actors at the user computing device, cause the food item to be moved for outbound shipment to a food processing plant.

The disclosed technology can provide one or more advantages. For example, the disclosed technology can provide a non-destructive way to identify ripeness of food items, such as fruits and produce. Since the food items may be non-destructively tested in real-time, this can reduce business losses and increase profits. After all, more unaltered food items can be sold to consumers, and such food items can be sold to consumers when they are a preferred ripeness.

Destructive measurements can be used to generate a ripeness metric and models without compromising or invading food items that are to be sold to consumers. Data can be collected using a variety of tools, such as by puncturing skin or flesh of the food items. This destructive data can be used to determine a ripeness metric. The models described herein can be trained to predict the ripeness metric using non-destructive data, such as spectra data. Destructive techniques may not be used in real-time to determine food item ripeness. Instead, spectrometer data can be collected in real-time and used with the model to more accurately determine food item ripeness levels. In some implementations, increases in ripeness detection accuracy can be achieved by training the model with training data obtained by both non-destructive and destructive measurements of historical food items. Accuracy in ripeness predictions can therefore be improved without destroying the food items that are sold to consumers.

The disclosed technology can also provide for a more accurate determination of food item ripeness. The models used to non-destructively determine ripeness can be trained on a variety of different data points, data sets, and inputs. The more data fed into the models, the more robust the models can become. More robust models can be advantageous to detect ripeness levels with more accuracy and without invading food items in real-time.

The disclosed technology can also be advantageous to improve supply chain management. Since food item ripeness can be determined more accurately and non-destructively, modifications to the supply chain can be appropriately made. The disclosed technology can detect subtle changes in suitability of consumption for food items based on changes in ripeness or firmness that may otherwise go undetected (or may otherwise be detected using destructive techniques). In such instances, a detected change in food item suitability for consumption can cause a modification in a distribution schedule for one or more food items. As an example, if certain produce is determined to be overripe, it may be disadvantageous to sell the produce in grocery stores to consumers. This produce may sell for a lower price and/or consumers may not purchase the produce. Therefore, the supply chain can be modified as soon as the ripeness of the produce is determined such that the produce can be moved to a food processing plant for processing. As another example, if some produce is determined to be ripe, it can be advantageous to move this produce to grocery stores for purchase by consumers. This produce may even be sold at a higher price based on whether the produce is a preferred ripeness. These supply chain modifications can be determined in real-time based on determined food item ripeness levels. As a result, the supply chain can become more efficient and modifications can be more accurately made in advance and without destructing the food items.

The disclosed technology can also use spectroscopic techniques, such as visible-NIR technology, to infer food item (e.g., fruit, produce) ripeness beyond a sensitive range of a durometer. Spectroscopic techniques can be advantageous to remove user-dependent measurement errors that can occur with the durometer. After all, spectroscopic techniques are non-invasive, non-destructive. Spectroscopic techniques can also be more easily scaled and automated. As such, this can provide a process improvement for scientists and other stakeholders in the supply chain by freeing up their time for other projects and providing more reliable and consistent data. NIR measurements can provide greater quantities of data that can be used to develop different models to infer other fruit quality metrics. NIR measurements can then be used in real-time to non-destructively determine food item ripeness.

As another example, the disclosed technology can provide for faster ripeness determination or prediction techniques. The disclosed technology can include an NIR spectrometer device that can include imaging and computing/processing capabilities. Therefore, spectrometer data can be captured and processed using the model(s) described herein at the NIR spectrometer device itself. Food item ripeness can be determined and outputted more quickly with edge computing at the NIR spectrometer device.

These and other innovative aspects of the present disclosure are described herein in the drawings, detailed description, and in the claims.

### BRIEF DESCRIPTION OF THE DRAWINGS

FIG. 1A is a conceptual diagram of generating a model for determining food item ripeness.
FIG. 1B is a conceptual diagram of determining food item ripeness in real-time.
FIG. 1C is a conceptual diagram of an example system for non-destructively determining whether a food item is suitable for consumption based on output data generated by the model.
FIG. 2 is a flowchart of a process for determining firmness levels of produce.
FIG. 3A is a flowchart of a process for generating the model for determining food item ripeness.
FIG. 3B is a flowchart of another process for generating a model for determining food item ripeness.
FIG. 3C illustrates firmness metrics that can be determined using data collected for training a model to determine food item ripeness.
FIG. 3D illustrates a firmness metric that can be predicted by a model trained to determine food item ripeness.
FIGS. 4A-B is a flowchart of a process for determining food item ripeness using the model in real-time.
FIG. 5 is another flowchart of a process for non-destructively determining whether the food item is ready for consumption using the model.
FIGS. 6A-B is a flowchart of a process for non-destructively determining whether a batch of food items are ready for consumption using the model.
FIG. 7 is a graphical depiction of determining food item ripeness using the model described herein.
FIG. 8 is a block diagram of system components that can be used to implement the systems, methods, and techniques described herein.

### DETAILED DESCRIPTION

The present disclosure is directed towards systems, methods, and techniques for determining whether a food item is suitable for consumption without destroying the food item. A ripeness metric can be engineered based on obtaining destructive measurements of test food items. A machine learning model can then be developed and trained to determine the ripeness metric using non-destructive measurements, such as spectra data from a spectrometer. In real-time, non-destructive measurements can be collected about a food item. The collected measurements can be inputted into the model to determine or predict the engineered ripeness metric (e.g., firmness, consumption suitability level) for the particular food item without invading or otherwise destroying the food item.

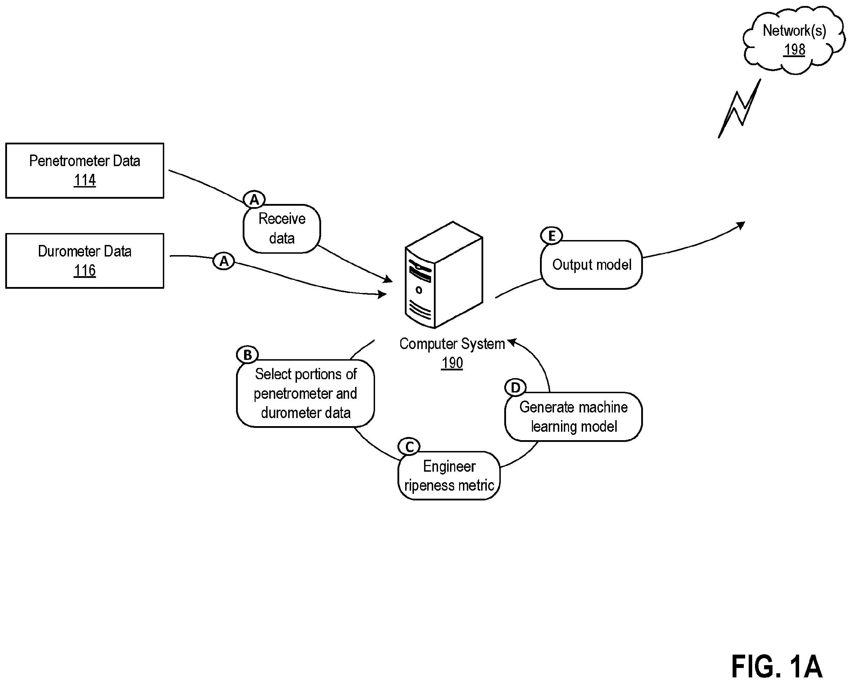

Referring to the figures, FIG. 1A is a conceptual diagram of generating a model for determining food item ripeness. As described herein, generating the model can include engineering a ripeness metric using destructive, invasive measurements. A computer system 190 can communicate with one or more components, systems, and/or devices described throughout this disclosure via network(s) 198. The computer system 190 can receive penetrometer data 114 and durometer data 116(A). The data 114 and 116 can be received from one or more devices.

For example, the penetrometer data 114 can be received from a penetrometer that is used on a test food item. The penetrometer can be used to determine firmness of the food item when the food item is hard. The penetrometer can be used to slice a layer of skin from the food item and/or to puncture through the skin of the food item. The penetrometer can measure a force that can be used to push the penetrometer probe through the skin of the food item. Thus, the penetrometer data 114 can be a destructive measurement used in determining the ripeness metric.

The durometer data 116 can be received from a durometer that is used on the test food item or other test food items. The durometer can include a thimble that can be pressed into the food item. The durometer can be used to determine firmness of the food item when the food item is soft. The durometer can measure a resistance force when a user presses the thimble into the food item. The durometer data 116 can be another destructive measurement used to engineer the ripeness metric.

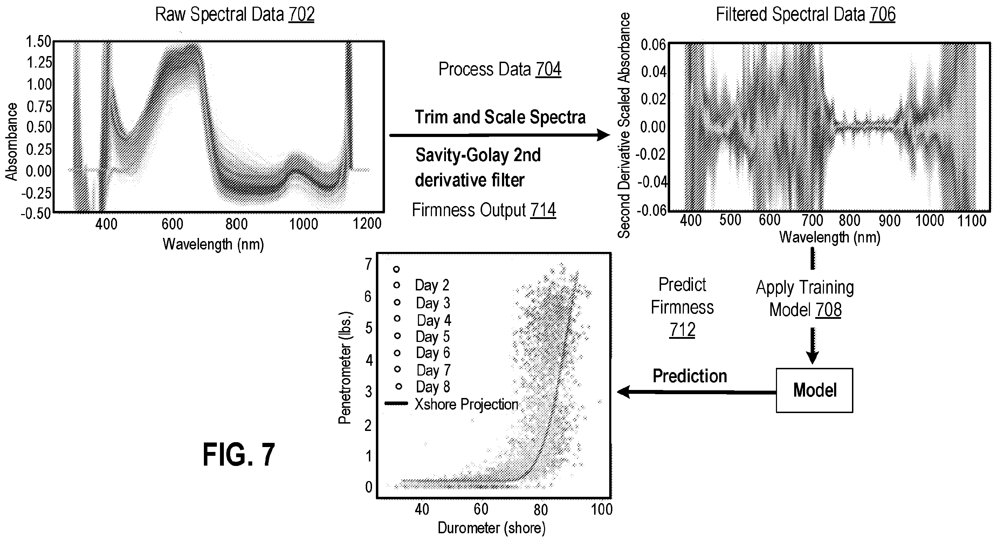

Using the received data, the computer system 190 can select portions of the penetrometer data 114 and the durometer data 116 to use for engineering the ripeness metric (B). As described herein, the data 114 and 116 can be graphed. The graph can be flattened using linear regression techniques. At some point, as shown by the graph (e.g., refer to FIG. 7), only durometer data 116 can be used to accurately predict food item firmness. Moreover, at other times, only penetrometer data 114 can be used to accurately predict the firmness. Thus, certain penetrometer and durometer values 114 and 116 can be selected around an inflection point in the graph. The selected values can then be modeled with wavelength or other spectral information .

The computer system 190 can then engineer the ripeness metric (C). As described herein, the model can be generated using the selected penetrometer data values 114 and the selected durometer data values 116.

The computer system 190 can then generate a machine learning model (D). The model can be generated to predict or determine the ripeness metric for food items of a same type in real-time. As described herein, the model can be used in real-time to predict the ripeness metric non-destructively. Moreover, in some implementations, the computer system 190 can generate more than one machine learning model. The one or more models can be trained to learn how to map spectra of food items to the engineered ripeness metric. Thus, the models can correlate different wavelengths with firmness changes (e.g., becoming less firm over time) to non-destructively predict ripeness of food items in real-time.

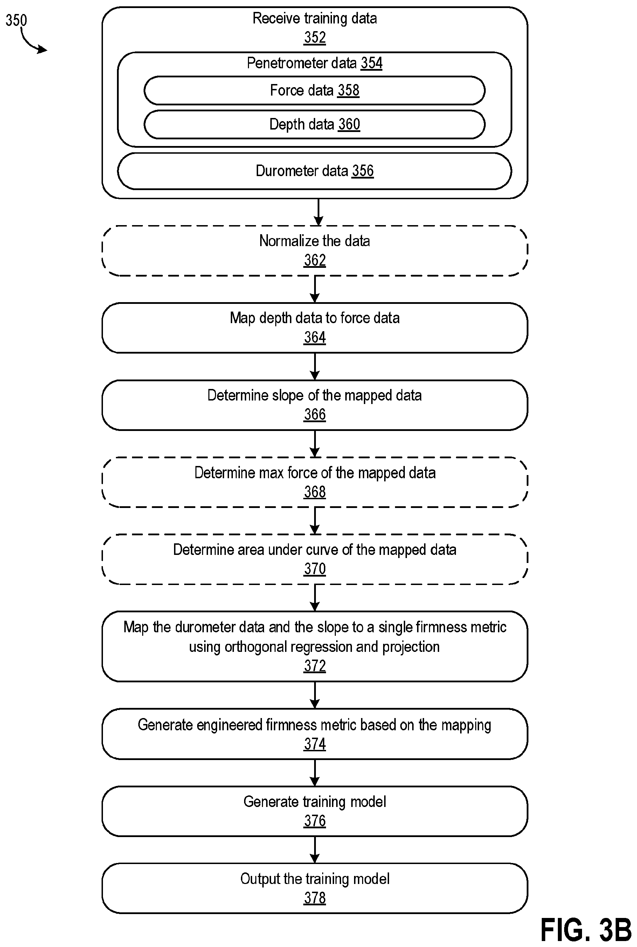

In some implementations, a slope of the penetrometer values 114 can indicate firmness, or ripeness, of the food item. Therefore, the computer system 190 can generate a model that predicts the slope from spectra data to determine the engineered ripeness metric for the food item. During training, the computer system 190 can collect penetrometer values 114, which can be graphed into a full firmness curve accounting for force (measured in pounds, lb, or grams) and depth (measured in millimeters, mm). The model can be trained to determine a slope of the firmness curve. The slope of penetrometer values 114 can accurately indicate a firmness metric, or the engineered ripeness metric, for some types of fruit, such as harder fruit. In some implementations, to account for a variety of different types of fruit (e.g., hard and soft fruit), the model can be trained using both the penetrometer values 114 and the durometer values 116. The values 114 and 116 can be normalized. Orthogonal regression and projection can be used to map the normalized durometer values 116 to the slope of the normalized penetrometer values 114 to generate a complete and representative firmness curve. The model can then be trained to predict the slope of the normalized penetrometer values 114 using spectra data received during runtime. Refer to FIGS. 3B-D for additional discussion.

Once the model is generated, the model can be outputted (E). Outputting the model can include storing it in a database or similar data store (e.g., cloud storage) for subsequent and/or future use. Outputting the model can also include presenting or providing the model to another computing device for real-time use. The outputted model can therefore be used to non-destructively determine ripeness levels of food items in real-time.

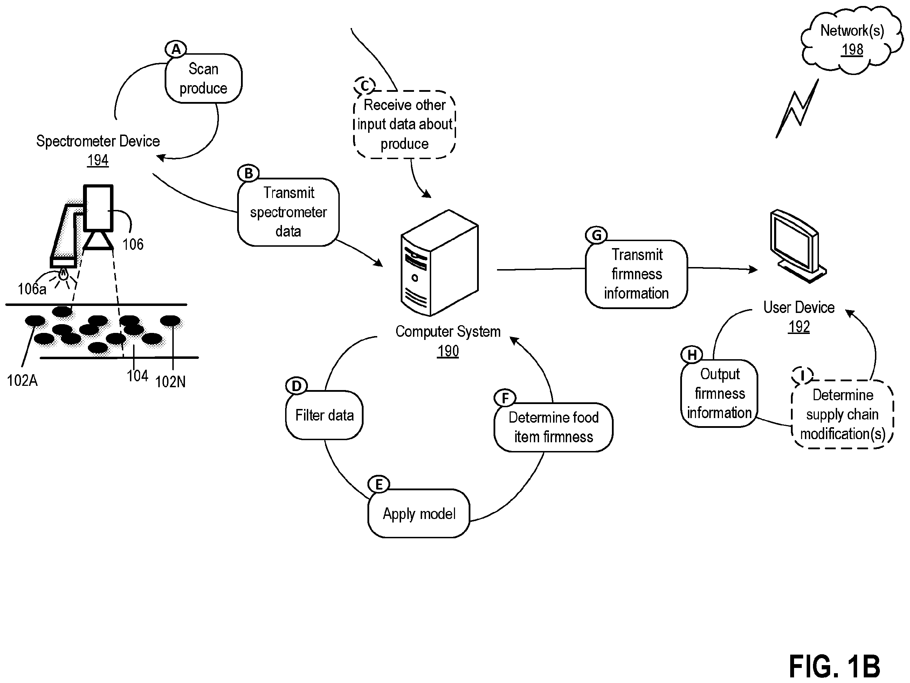

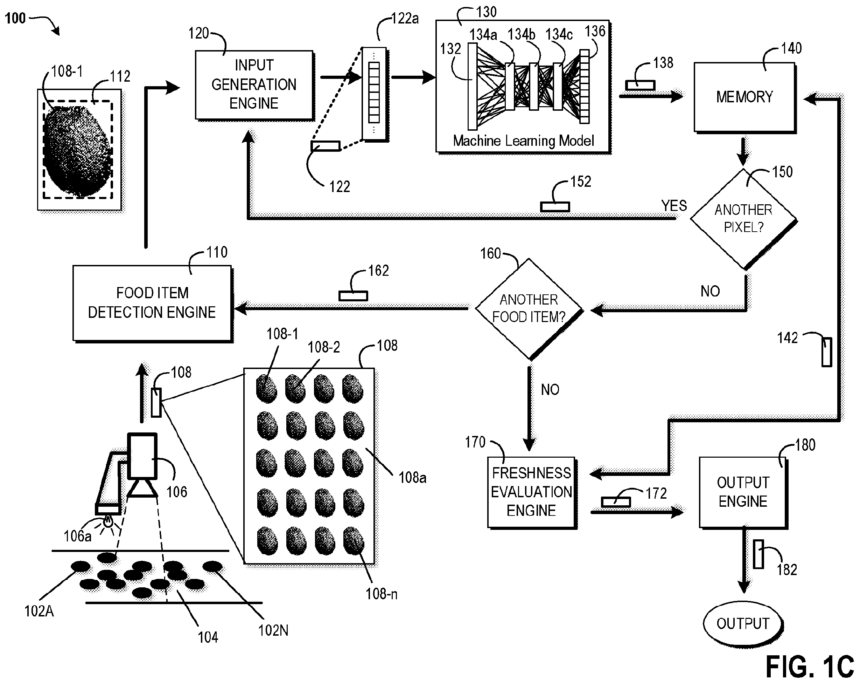

FIG. 1B is a conceptual diagram of determining food item ripeness in real-time. As described herein, ripeness can be determined using the model described in FIG. 1A. Ripeness can be determined in real-time using non-destructive measurements (e.g., spectrometer data) and techniques. The computer system 190 can communicate (e.g., wired and/or wirelessly) with a user device 192 and a spectrometer device 194 via the network(s) 198. The spectrometer device 194, which has an image sensor 106 and a light source 106a, is described in further detail in FIG. 1C. The user device 192 can be a computing device such as a mobile phone, cellphone, laptop, computer, and/ or tablet. The user device 192 can be used by a supply chain worker or other relevant stakeholder.

In some implementations, the spectrometer device 194, the computer system 190, and the user device 192 can be one computing system. In other implementations, one or more of the devices described herein can be one computing system.

As shown in FIG. 1B, produce 102A-N can be placed on a conveyor belt 104. In some implementations, the produce 102A-N can be on pallets, in boxes, or in other containers for transport. The conveyor belt 104 can be within a warehouse, such as a cold storage facility. The spectrometer device 194 can be configured to scan the produce 102A-N as it moves along the conveyor belt 104 (A).

In some implementations, the produce 102A-N can be scanned as soon as it enters the warehouse. Scanning at this time can be advantageous to make more immediate changes to a supply change based on the determined ripeness level(s) of the produce 102A-N. In some implementations, the produce 102A-N can be scanned at different times while being moved on the conveyor belt 104 through the warehouse. For example, the produce 102A-N can also be scanned before the produce 102A-N is moved out from the warehouse. In other words, determining the ripeness of the produce 102A-N at this stage can be advantageous to determine whether the produce 102A-N should be directed to stores and end-consumers or whether the produce 102A-N should be directed to food processing plants.

The spectrometer device 194 can then transmit the spectrometer data to the computer system 190 (B). The computer system 190 can optionally receive other input data about the scanned produce 102A-N (C). For example, the computer system 190 can retrieve, from a database, historic information about the produce 102A-N. The historic information can include a place of origin of the produce 102A-N, typical ripening conditions for the produce 102A-N, and/or preferred climate conditions for the produce 102A-N. The other input data can also include any other historic or relevant information associated with the produce 102A-N as described throughout this disclosure.

The computer system 190 can then filter the received data (D) (e.g., refer to FIG. 7). The model can be applied to the filtered data (E). Using the model, the computer system 190 can determine food item firmness (F). The computer system 190 can then transmit the firmness information to the user device 192 (G). The firmness information can be outputted at the user device 192 (H). Optionally, supply chain modifications can be determined at the user device 192 (I). These modifications can be automatically determined by the user device 192 and/or one or more other computing systems in communication with the user device 192. The modifications can also be determined by a user at the user device 192. As described throughout this disclosure, the modifications can include routing produce 102A-N for outbound to a processing plant when the produce 102A-N is not as firm or ripe and routing produce 102A-N for outbound to stores and customers when the produce 102A-N is a certain preferred firmness and/or ripeness.

FIG. 1C is a conceptual diagram of an example system 100 for non-destructively determining whether a food item is suitable for consumption based on output data generated by the model. The system 100 can include an image sensor 106, a food item detection engine 110, an input generation engine 120, a machine learning model 130, a memory 140, a first program logic engine 150, a second program logic engine 160, a freshness evaluation engine 170, and an output engine 180. An "engine" can include one or more software modules, one or more hardware modules, or any combination thereof.

The image sensor 106 can used to generate data 108 that represents attributes of food items 102A-N, where N can be any positive integer number greater than 0 and represents the number of food items 102 on a conveyor belt 104. In the example of FIG. 1, the image sensor 106 can be arranged in a manner that enables the image sensor 106 to capture image data that represents one or more images of the food items 102A-N as the food items 102A-N move along the conveyor belt 104. In some implementations, the sensor 106 can include one or more hyperspectral sensors configured to capture hyperspectral data that represents features of the food items 102A-N. In such implementations, each pixel of the hyperspectral image can correspond to a spectrum of infrared or ultraviolet light associated with corresponding food item imaged by the hyperspectral sensor. In some implementations, a point spectrometer may be used as the image sensor 106. The point spectrometer can generate a single output representative of a spectrum of infrared or electromagnetic light associated with an imaged region of the conveyor 104. In some implementations, the sensor 106 can also be a low-resolution digital camera (e.g., 5M or less), a high-resolution digital camera (e.g., 5MP or more), or the like.

In some implementations, the sensor 106 can include multiple sensors positioned at multiple angles relative to one or more food items 102A-N. For example, the sensor 106 can include a first camera and at least one additional camera that each capture images of the food items 102A-N from different perspective angles. In such configurations, the one or more additional cameras can be used to generate image data based on different or additional wavelengths of light than the wavelengths of light captured by the first camera and used, by the first camera, to generate image data representative of the food items 102A-N. In general, any set of wavelengths of light can be obtained by the sensor 106.

Each particular camera of the one or more cameras can be configured to detect the different or additional wavelengths of light in a number of different ways. For example, in some implementations, different sensors can be used in different cameras in order to detect different or additional wavelengths of light. Alternatively, or in addition, each of the one or more cameras can be positioned at different heights, different angles, or the like relative to each other to capture different wavelengths of light. In some implementations, one or more cameras can be positioned, at least in part, to capture portions of one or more food items 102A-N that may be obscured from a view of the first camera.

In some implementations, one or more light sources 106a can be used to illuminate the food items 102A-N. As described in reference to FIG. 1B, the light source 106a and the sensor 106 can comprise a spectrometer device. The light source 106a can illuminate the food items 102A-N so that image sensor 102, such as a hyperspectral image sensor or other spectrometer sensor, can generate images that capture light reflecting off the food items 102A-N. The light source 106a can include one or more light sources that each produce the same or different electromagnetic radiation. In this example, the light source 106a is depicted as being affixed to the image sensor 106. In some implementations, the light source 106a can be positioned in one or more locations proximate the image sensor 106 in order to illuminate the food items 102A-N before, or during, capture of reflected light by the image sensor 106 to generate the image data 108. In some implementations, the one or more light sources can be selected based on a frequency of electromagnetic radiation output by the one or more light sources. For example, in some implementations, the light source 106a can be a halogen light source. Alternatively, or in addition, the one or more light sources 106a can be a diode or series of diodes of broadband light-emitting diodes (LEDs) that can be used to provide light across visible wavelength spectrum, near infrared wavelength spectrum, electromagnetic spectrum, or any other spectrum of light. In general, any light source can be used to provide any type of light for the image sensor 106.

In some implementations, the one or more light sources 106a or a control unit of the one or more light sources 106a can be communicably connected to the image sensor 106 or a control unit of the image sensor 106. For example, the image sensor 106 or the control unit of the image sensor 106 can send a signal to the one or more light sources 106a or a control unit of the one or more light sources 106a that cause the one or more light sources 106a to illuminate one or more of the food items 102A-N with one or more wavelengths of light at a predetermined power and at a predetermined time. In some implementations, the predetermined time can be a predetermined amount of time before, or during, capturing of the image data 108 by the image sensor 106 so that the image sensor 106 can capture the image data 108 of the food items 102AN when the food items 102A-N are illuminated.

In some implementations, the control unit of the image sensor 106 can include one or more computers or computing systems that can send one or more signals to the image sensor 106 that cause the image sensor 106 to capture image data 108. The image data 108 can include one or more images. Such images can include one or more hyperspectral images when the image sensor 106 includes a hyperspectral image sensor. The images can also be data generated by a spectrometer, digital images less than 5 MP, digital images of 5 MP, or digital images greater than 5 MP. In some implementations, the control unit of the one or more light sources 106a can includes one or more computers that send one or more signals to the one or more light sources 106a that cause the one or more light sources 106a to output light.

In some implementations, the light source 106a can be configured to output a particular wavelength of light based on a type of food item that is evaluated by the system 100 to determine the food item's suitability for consumption. For example, if the food items 102A-N include avocados and a machine learning model 130 has been trained to determine a consumption suitability attribute of avocado firmness (e.g., refer to FIG. 1A for generating the model), the control unit of the light source 106a can be configured to instruct the light source 106a to output light having a wavelength range of 534nm to 942 nm, as such wavelengths of light can be useful in generating, by the image sensor 106, image data 108 having optimal features for the machine learning model 130 to detect a level of firmness of an avocado.

As another example, if the food items 102A-N include avocados and the machine learning model 130 has been trained to determine a consumption suitability attribute of avocado dry matter, the control unit of the light source 106a can be configured to instruct the light source 106a to output light having a wavelength range of 690nm to 912nm, as such wavelengths of light can be useful, by the image sensor 106 to capture image data 108 having optimal features for the machine learning model 130 to detect a level of dry matter of the avocado.

As another example, if the food items 102A-N include apples, the machine learning model 130 can be trained to determine a consumption suitability attribute of apple firmness, the control unit of the light source 106a can be configured to instruct the light source 106a to output light having a wavelength range of 672nm to 948nm, since such wavelengths of light can be useful to generate image data 108 having optimal features for the machine learning model 130 to detect a level of firmness of an apple.

As demonstrated by the examples herein, light output by the light source 106a can be customized based on the type of food items 102A-N to enable the image sensors 106 to generate image data 108 having optimal features that yield accurate inferences, via the machine learning model 130, to detect suitability for consumption (e.g., ripeness). Any wavelength of light can also be selected for one or more different food types.

In some implementations, the control unit of the image sensor 106 or light source 106a can dynamically configure the light source 106a to output a particular wavelength of light based on newly detected food items. In some implementations, the dynamic configuration of the light source 106a can occur in response to an instruction received from a user device. For example, a user can configure the light source 106a for a particular type of food item 102A-N. In such implementations, a user of a user device can input a command that causes the user device to generate and transmit an instruction to the controller of the image sensor106 or light source 106a that causes the light source 106a to output a particular wavelength of light.

In some implementations, the system 100 can automatically determine, without user interaction, that a particular type of food item is to be evaluated by the system 100 and thus automatically configure the light source 106a. For example, the system 100 can include another one or more cameras at an earlier stage along the conveyor belt 104 or in one or more other locations along the supply chain. These one or more other cameras can capture one or more images of the food items 102A-N placed onto the conveyor belt 104, analyze the one or more images of the food items 102A-N, and determine, based on the analyzed images, a particular type food item to be evaluated by the system 100. In such implementations, the one or more other cameras can be communicably coupled to a communication unit that can communicate, to a control unit of the image sensor 106 or light source 106a the type of food item to be evaluated by the system 100. Then, based on the received communication, the control unit of the image sensor 106 or light source 106a can dynamically configure the light source 106a in a manner that causes the light source 106a to output wavelengths of light that fall within an optimal range of light wavelength for the detected type of food items.

The image data 108 generated by the image sensor 106 can be provided as an input to a food item detection engine 110. In some implementations, the image sensor 106 can directly provide the image data 108 to a software or hardware engine configured to process the image data 108. In other implementations, the image sensor 106 can store the image data 108 in a memory device such as memory 140 and then the food item detection engine 110 can access the memory device 140 to obtain and process the image data 108.

The food item detection engine 110 can obtain the image data 108. The obtained image data 108 can include at least first image data 108-1 to 108-n and a second image 108a, where n is any positive integer greater than zero and corresponds to a number of food items depicted in the image data 108. The first image data 108-1 to 108-n can include a depiction of n food items and the second image data 108a can corresponds to a portion of the environment such as the conveyor belt 104 or a portion of a processing facility where the food items are being processed. The portion of the processing facility can be depicted in the image data 108 during image capture of a portion of the food items 102A-N on the conveyor belt 104. The food item detection engine 110 can process the obtained image data 108 and extract a portion 112 of the first image data 108-1 to 108-n that corresponds to a first food item 108-1.

In some implementations, the food item detection engine 110 can use one or more object recognition algorithms, methods, or techniques to recognize portions of the image data 108 that correspond to features of food items 108-1 to 108-n depicted in the image 108. For example, as shown in FIG. 1C, the food items 102A-N can be avocadoes. In this example, the food item detection engine 110 can be trained on a plurality of images of avocadoes such that the food item detection engine 110 can determine, based on an input image, whether or not the input image includes a representation of an avocado and where the representation of the avocado appears within the input image 108. The food item detection engine 110 can also be used to detect other food items such as citrus fruits, mangos, apples, berries, stone fruits, tomatoes, meat, and/or vegetables. Though this list provides an example of food items that fall within the scope of the present disclosure, the present disclosure is not limited to these food items.

In some implementations, a coordinate system can be used to determine a location of the representation of a food item, e.g., 108-1, in the image data 108. For example, one or more numerical values, such as x and y values in an x and y coordinate system, can be used to represent the location of the food item 108-1 in the image data 108. Subsequent processing steps can use the numerical values that represent the location of the food item 108-1 and determine, based on the numerical values, where in a given image, the food item 108-1 is located.

In some implementations, the food item detection engine 110 can include a network of one or more machine-learning models. In such implementations, the network of one or more machine-learning models of the food item detection engine 110 can be trained based on a training data set of a particular type of food item to detect. In the example of FIG. 1C, the food item detection engine 110 can be trained on images of avocados such that the food item detection engine 110 can detect the location and appearance of avocadoes within a given input image.

The food item detection engine 110 can output image data 112 that was extracted from the image data 108. The image data 112 can include a portion of the image data 110 that corresponds to a first food item 108-1. The image data 112 can be provided as input to an input generation engine 120. In some implementations, the food item detection engine 110 can directly provide the image data 112 to a software or hardware engine configured to process the image data 112 such as the input generation engine 120. In other implementations, the food item detection engine 110 can store the image data 112 in a memory device such as memory 140 and then the food item detection engine 110 can access the memory device 140 to obtain and process the image data 112.

The input generation engine 120 can generate data 122 for input to the machine learning model 130. The generated input data 122 can include a vector 122a that numerically represents features of the extracted image data 112. For example, the vector 122a can include a plurality of fields that each correspond to a pixel of the image data 112. The input generation engine 120 can determine a numerical value for each of the fields that describes the corresponding pixel of the image data 112. The determined numerical values for each of the fields can be used to encode the features of the image data 112 into a generated vector 122a. The generated vector 122a, which numerically represents the image data 112, can be provided as an input to the machine learning model 130.

The machine learning model 130 can include a deep neural network having an input layer 132 for receiving input data, one or more hidden layers 134a, 134b, 134c for processing the input data received via the input layer 132, and an output layer 136 for providing output data. Each hidden layer 134a, 134b, 134c can include one or more weights or other parameters. The weights or other parameters of each respective hidden layer 134a, 134b, 134c can be adjusted so that the trained deep machine learning model 130 can produce a desired target vector corresponding to each set of training data. The output of each hidden layer 134a, 134b, 134c can include an activation vector. The activation vector output by each respective hidden layer can be propagated through subsequent layers of the deep neural network and used by the output layer to produce output data. For example, the output layer 136 can perform additional computations of a received activation vector from the final hidden layer 134c in order to generate neural network output data 138.

As described throughout this disclosure, the machine learning model 130 can be trained to generate output data 138 that is indicative of a level of consumption suitability of a food item represented by the input data 122. The machine learning model 130 can be trained using a plurality of training data items. For example, as described in reference to FIG. 1A, the model 130 can be trained using destructive measurements (e.g., penetrometer data) in combination with other metrics, such as durometer data, spectrometer data and historic information about a food item. Each training data item of the plurality of training data items can also include (i) a training image of a food item, and (ii) a label that describes food item classification for the food item depicted by the training image. The food item classification for each training data item can be a classification determined based on historical data collected from real-world food items to which a particular training data item corresponds. This historical data collected for the food items can include first food item suitability data obtained using a non-destructive measurement of the food item, such as a durometer reading, second food item suitability data obtained using a destructive measurement of the food item, such as a penetrometer reading, or both.

Generating the model using a variety of input metrics, both destructive and non-destructive, can be advantageous to predict ripeness or other consumption suitability levels in a non-destructive manner. After all, the machine learning model 130 can be trained to predict a level of consumption suitability for a food item in real-time and without destroying the food item, but while using layers 134a, 134b, 134c of the machine learning model 130 that have weighted parameters trained to recognize subsequent image data of food items having similar features to training images of food items having particular measurements obtained using destructive processes. Thus, the machine learning model 130 can use destructive measurements of a food item to evaluate a consumption suitability attribute of a food item, such as a firmness or ripeness, without actually destroying the food item.

Still referring to FIG. 1C, a training system can include the machine learning model 130 and a database having a plurality of labeled training data items. Each training data item can be an image of a food item and be labeled with (a) a durometer reading measured from the actual food item, (b) a penetrometer reading obtained from the actual food item, and/or (c) a spectrometer reading obtained from the actual food item. During training of the machine learning model 130, a training image can be obtained from a training image database and provided as an input to the machine learning model 130. The machine learning model 130 can process the training image and generate output data corresponding to (a) a predicted durometer reading for the food item in the training image, (b) a predicted penetrometer reading for the food item in the training image, and/or (c) a predicted spectrometer reading for the food item in the training image. The output generated by the machine learning model 130 can then be compared to the labeled readings. The parameters of the machine learning model 130 can be adjusted based on a difference between the generated output data and the labeled readings. This process can iteratively continue for each of a plurality of training items in the database of training items until a loss function such as a regression loss function is optimized.

The output data that the machine learning model 130 is trained to produce a non-destructive component, a destructive component, or both. This output can be referred to as a level of consumption suitability, ripeness, and/or firmness. In some implementations, this output can be separate values and can be generated, for example, by the trained machine learning model 130 processing input data, such as input data 122, through each layer of the trained machine learning model 130 in order to classify the input data 122 into a non-destructive measurement and a destructive measurement classification.

In some implementations, the output data 138 can be indicative of a predicted non-destructive measurement and a predictive destructive measurement. In some implementations, the machine learning model 130 can be trained to predict a single consumption suitability value indicative of a relationship between the non-destructive measurement and the destructive measurement. For example, in such implementations, the machine learning model 130 can be trained to predict a value representative of a slope of the destructive measurement (e.g., penetrometer measurement value) over a slope of the non-destructive measurement (e.g., durometer measurement value).

Still referring to the example in FIG. 1C, the machine learning model 130 can process the input data 122 through each of the layers 132, 134a, 134b, 134c, 136 of the trained machine learning model 130 and generate output data 138. The output data 138 can represent a consumption suitability level for the food item depicted by the image data 112 and represented by input data 122. The trained machine learning model 130 can store the output data 138 in the memory 140 and the system 100 can make a determinations as to how execution of the system 100 can proceed.

At 150, the system 100 can determine whether there is additional data to be processed through the trained machine learning model 130. This determination can be based on a type of image data 108 generated by the imaging sensor 106. In some implementations, for example, a point spectrometer can be used to generate the image data 108 and there may be additional data to process by the machine learning model 130.

In some implementations, the image data 108 can include hyperspectral image data. In such implementations, the input generation engine 120 can be configured to generate input data 122 and process the generated input data 122 through the trained machine learning model 130 for each pixel of the hyperspectral image data 108. In such implementations, the system 100 can determine at 150 whether another pixel of the image data 112 can be processed through the trained machine learning model 130. If the system 100 determines that another pixel of the image data 112 can be processed, the system 100 can instruct 152 the input generation engine 122 to generate a subsequent input data 122 based on another pixel of the image data 112. The subsequent input data 122 can also be processed through the machine learning model 130 to generate subsequent output data 138. The subsequent output data 138 can be stored in the memory 140. The system 100 can again determines at 150, whether additional data, such as another pixel of the image 112, can be processed. If the system 100 decides additional data can be processed, the aforementioned process can iteratively continue until a termination condition is triggered at 160 indicating that no more pixels of image data 112 can be processed. In some examples where a point spectrometer is used, this condition can terminate upon the first occurrence of 150.

The system 100 can continue evaluation of the food items 102A-N at 160 after the termination condition is triggered at 150. At 160, the system 100 can determine whether another food item is depicted in the image data 108. If another food item is depicted in the image 108, then the system 100 can instruct 162 the food item detection engine 110 to process the image data 108 and extract another image of another food item, such as a portion of the image data 108 corresponding to food item 108-2. Then, the system 100 can process subsequent image data 112 that corresponds to food item 108-2 through the system 100 as described above with respect to the image data 112 that depicts food item 108-1. The system 100 can continue execution of this process until each of n images of food items in image data 108 are processed.

Upon a subsequent execution of 160 after processing of each pixel of the image of the nth food item, the system 100 can terminate execution of the process described herein for evaluating food items at 160. At this point, the system 100 can instruct a freshness evaluation engine 170 to analyze the output data 138 generated and stored in the memory 140 for each pixel of each image of each food item in image data 108. For example, the freshness evaluation engine 170 can access an aggregate representation of data 142 stored in the memory 140 and analyzing the aggregate representation of the output data 138 across each of the pixels of each of the images.

In some implementations, the freshness evaluation engine 170 can determine an average level of consumption suitability across all, or a subset, of the output data 138 generated and stored in the memory 140. In such implementations, the average level of consumption suitability can be evaluated using a threshold value. If the average level of consumption suitability satisfies the predetermined threshold, then the system 100 can generate instructions 172 to an output engine 180 indicating that the food items depicted by the image data 108 are suitable for consumption. As another example, if the average level of consumption suitability does not satisfy the predetermined threshold, then the system 100 can generate instructions 172 to the output engine 180 indicating that the food items depicted by the image data 108 are not suitable for consumption.

In some implementations, satisfying the predetermined threshold can include a value that is greater than the threshold. In some implementations, a value may be held to satisfy a predetermined threshold if the value is less than the threshold. For example, in some implementations, both the value and a comparison operator can be negated. In such implementations, a same value that may otherwise be greater than the threshold can also be represented as being less than the threshold value. Similar operations can be performed for a mean of the output data 138, for a median of the output data 138, or the like.

As another example, the freshness evaluation engine 170 can evaluate the aggregate set of output data 138 stored in the memory 140 by analyzing a distribution of consumption suitability values across the complete set of consumption suitability values. In such implementations, the freshness evaluation engine 180 can determine whether the food items 108-1 to 108-n are suitable for consumption based on a manner in which the distribution of consumption suitability values adheres to or deviates from an expected distribution of consumption suitability values for food items that are known to be suitable for consumption. The freshness evaluation engine 170 can instruct 172 output engine 180 to generate and provide output data based on such distribution analysis.

The output engine 180 can receive instructions 172 from the freshness evaluation engine 170 that indicate level of suitability for consumption. In some implementations, the freshness evaluation engine 170 can determine that the food items 108-1 to 108-n are suitable for consumption, and the instructions 172 can instruct the system 100 to continue with a distribution plan for the food items 108-1 to 108-n. In some implementations, the freshness evaluation engine 170 can determine that the food items 108-1 to 108-n may not be suitable for consumption, and the instructions 172 can instruct the system 100 to discard the food items 108-1 to 108-n depicted by the image data 108. In yet other implementations, the freshness evaluation engine 170 can determine that the food items 108-1 to 108-n will become not suitable for consumption within a certain number of days. In such implementations, the freshness evaluation engine 170 can instruct 172 the output engine 180 to generate a change in the distribution plan for the food items 108-1 to 108-n. Such changes can include but is not limited to shipping the food items 108-1 to 108-n to a market that is geographically closer than a previously scheduled market so that the food items 108-1 to 108-n can be provided to consumers sooner. A change in the distribution plan for the food items 108-1 to 108-n can also include a transit modification that causes a delivery vehicle to be re-routed to get the food items 108-1 to 108-n to a refrigeration unit faster/sooner.

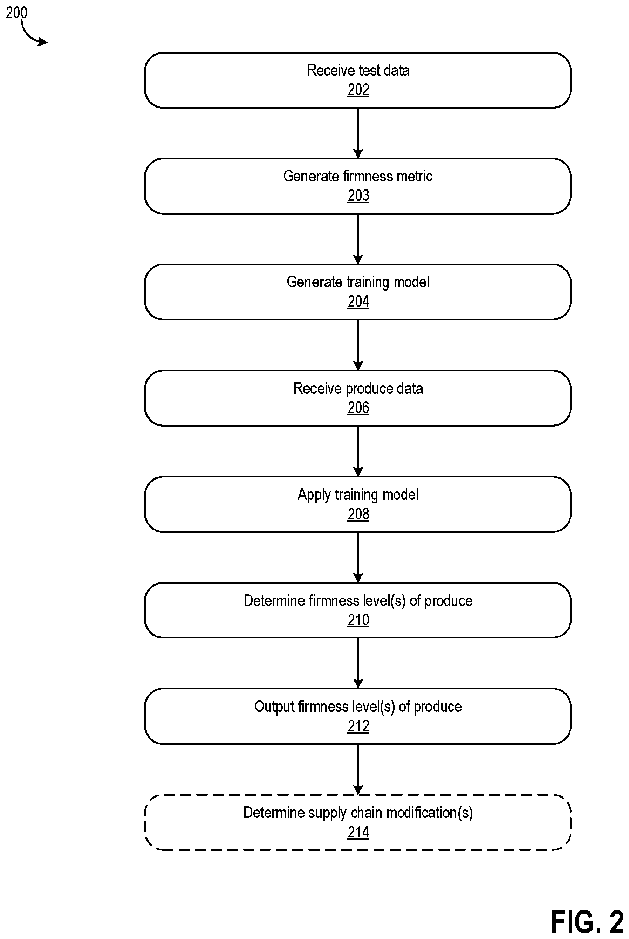

FIG. 2 is a flowchart of a process 200 for determining firmness levels of produce. The process 200 can be performed by one or more of the computing systems described herein. For example, the process 200 can be performed by the computer system 190, the user device 192, and/or the system 100 (e.g., refer to the FIGS. 1A-C). For simplicity and exemplary purposes, the process 200 is described from the perspective of the computer system 190.

Referring to the process 200, the computer system 190 can receive test data before run-time use (202). As described herein (e.g., refer to FIG. 1A), the test data can include destructive measurements for produce of a same food type. For example, the destructive measurements can include penetrometer and/or durometer data about one or more test produce in a batch. The penetrometer and/or durometer data can be captured by invasively slicing or puncturing the test produce. The data can be collected and inputted into the computer system 190.

In 203, the computer system 190 can generate a firmness metric (e.g., ripeness metric). As described herein, the firmness metric can be based on the destructive measurements received in 202. The test data described above can be used to build a ripeness curve, which can be used as a ground truth to train a non-destructive spectral model. One or more different firmness metrics can be generated for different type of produce and/or different ripeness characteristics. For example, a firmness metrics can be generated for detecting ripeness of berries. Test data for a berries ripeness curve can include color, brix, sugars, and pH. One or more of this test data can be destructively measured. Using this test data, a ripeness curve can be generated and used as a ground truth measurement to train a spectral model to predict ripeness of berries. A machine learning model can then be generated to measure the berry ripeness metric in real-time using non-destructive measurements. Thus, in real-time, spectral data can be non-destructively captured of berries. The spectral data can be run through the model to non-destructively predict ripeness of such berries.

The computer system 190 can then generate a model (204) (e.g., refer to FIG. 1A). This model can be used to non-destructively determine a firmness, ripeness, and/or level of consumption suitability for produce in real-time. The model can be generated using the firmness metric.

During run-time, the computer system 190 can receive produce data in 206. The produce data can be non-destructively captured. For example, the produce data can be spectral imaging data as described above (e.g., refer to FIGS. 1B-C). The spectral imaging data can be captured by a spectrometer and/or a hyperspectral imaging device, as described throughout this disclosure. The produce data can be captured in real-time as produce is moved along a conveyor belt or within one or more locations of a storage facility. Moreover, the produce data can optionally be filtered and/or processed (e.g., refine image data, remove noise from the image data, etc.).

The computer system 190 can apply the model in 208. As mentioned above, one or more models can be generated for different type of produce. The computer system 190 can identify what type of produce is captured in the produce data and then apply the appropriate model. Using the model, the produce data can be analyzed to determine a ripeness metric for that produce.

The firmness level(s) of the produce can be determined by the computer system 190 in 210. Thus, the computer system 190 can determine whether the produce is ripe and/or ready for human consumption based on application of the appropriate model. As described in reference to FIG. 1C, the produce can be determined as ripe where output from applying the model exceeds a predetermined threshold value. The produce can be determined as not yet ripe where output from applying the model is less than the predetermined threshold value. In some implementations, the computer system 190 can determine firmness level for one or more individual produce in a batch. In some implementations, the computer system 190 can determine an aggregate firmness level for the batch of produce.

The firmness level(s) of the produce can be outputted in 212. The output can be presented at the user device 192 described herein or at one or more other computing devices, such as mobile phones, cellphones, tablets, laptops, and/or computers. The output can indicate how ripe the produce is. The output can also indicate how unripe, how firm, and/or how overripe the produce is. The output can be presented as graphical depictions. The output can also be presented as numeric values. In some implementations, the output can be presented in a preferred format for the related supply chain.

Optionally and additionally, the computer system 190 can determine one or more supply chain modification(s) (214). For example, the computer system 190 can determine that the produce can be moved for outbound to grocery stores for immediate consumption by consumers. Where the produce is determined to be soft rather than firm (e.g., overripe), the computer system 190 can determine that the produce should be moved for outbound to food processing plants. The produce can then be used in production of processed foods. As another example, where the produce is determined to be at an optimal firmness and/or ripeness, the computer system 190 can determine that the produce should be moved for outbound to grocery stores that are geographically closest to a current location of the produce (e.g., a storage facility). Therefore, the produce can be immediately put on shelves to be purchased and consumed by consumers. On the other hand, where the produce is determined to be at an optimal firmness and will remain at the optimal firmness for an extended period of time, the computer system 190 can determine that the produce can be moved for outbound to grocery stores that are geographically farther away from the current location of the produce. After all, a longer transport time may not negatively impact the firmness of the produce since the produce may still be firm when it arrives at the grocery stores. One or more other supply chain modifications can be determined in 214. Moreover, in some implementations, one or more supply chain modifications can be suggested by the computer system 190 and/or a user at the computer system 190 and/or the user device 192.

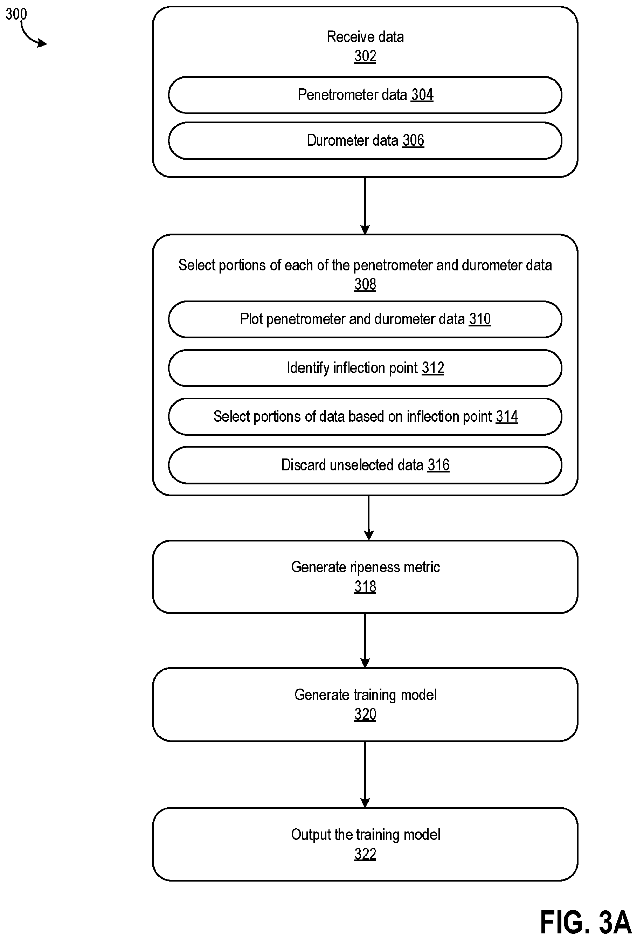

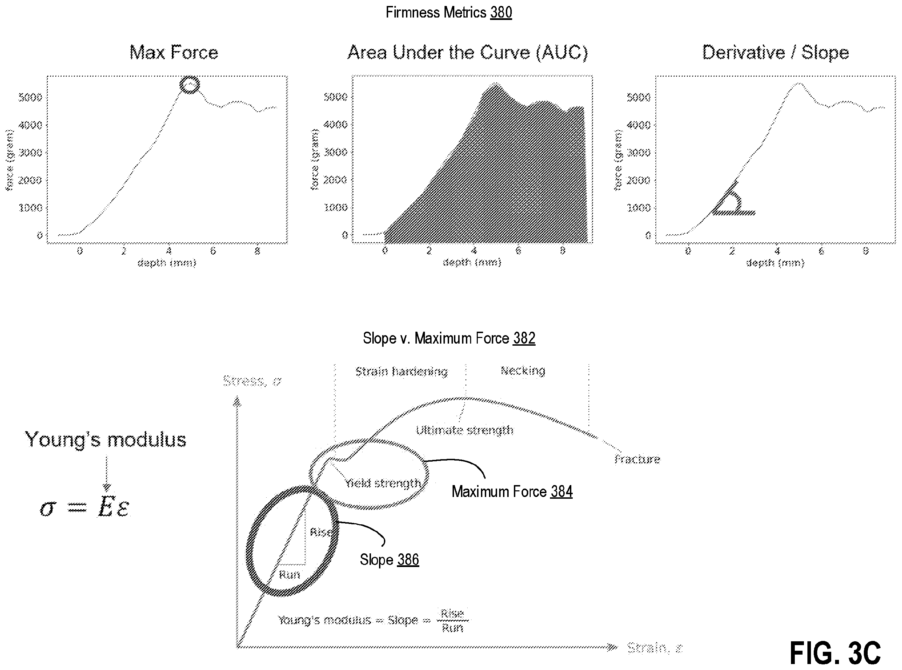

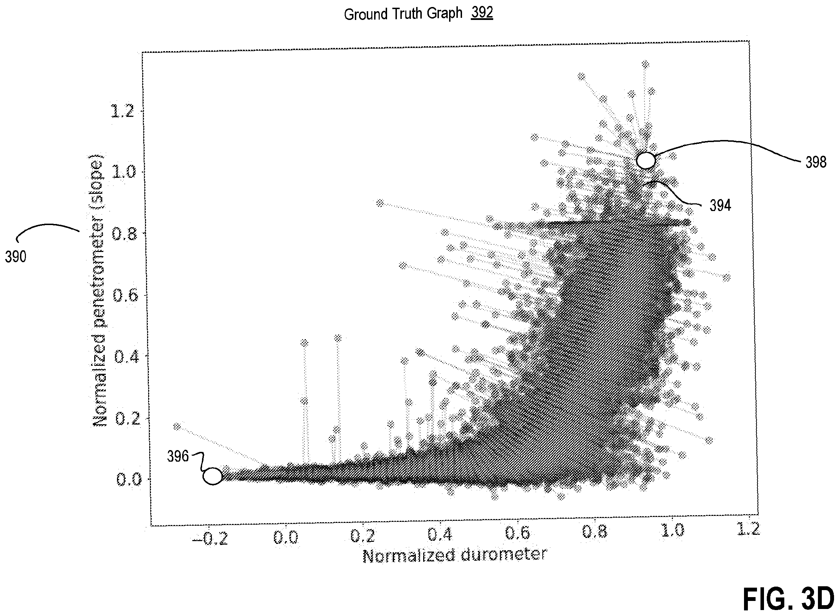

FIG. 3 is a flowchart of a process 300 for generating the model for determining food item ripeness. The process 300 can be performed by one or more of the computing systems described herein. For example, the process 300 can be performed by the computer system 190, the user device 192, and/or the system 100 (e.g., refer to the FIGS. 1A-C). For simplicity and exemplary purposes, the process 300 is described from the perspective of the computing system 190.

Referring to the process 300, the computer system can receive data in 302. As described throughout this disclosure, the computer system can receive penetrometer data 304 and durometer data 306. Data collection in 302 can be performed on one or more test food items, such as avocados. For example, an avocado can be scanned twice with the NIR device, receive 3 durometer measurements, and receive 2 penetrometer measurements. Any other set of data collection can occur. Groups of 18-35 avocados, or other group sizes, can be processed per day until each group is at a stage ready to be eaten (e.g., <60 shore). As a result, firmness changes associated with two commonly used firmness measurements (penetrometer and durometer) can be captured and associated with spectral information to capture firmness changes throughout the avocado ripening cycle.

The computer system can then select portions of each of the penetrometer data and the durometer data in 308. A nonlinear projection can be fit to transform durometer and penetrometer measurements into a single variable, which can be termed an Extended Shore Projection.

The computer system can plot the penetrometer and durometer data in 310. The Extended Shore Projection line can be fit using orthogonal regression in order to minimize Euclidean distance from a parameterized line to each penetrometer and/or durometer data point. While durometer data can have maximum values around 85-90 shore, this nonlinear projection can translate initial ripening behavior, which may initially only be detected with penetrometer data, into an extension of a shore measurement up to ~120 extended shore. This metric allows for capturing more range in initial firmness of avocados or other produce, and can discriminate an actual stage in the ripening lifecycle between different sets of unripe fruit.

The computer system can then identify an inflection point in 312 (e.g., refer to FIG. 7). At some point, only durometer values or only penetrometer values may be used to develop a ripeness metric. This is because at the inflection point, it can be uncertain whether a food item is either too firm or too soft. Thus, as an example, before the inflection point, penetrometer values can be used for modeling with wavelength or spectral imaging information. After the inflection point, durometer values can be used and modeled with wavelength or spectral imaging information.

Once the inflection point is identified, the computer system can select portions of data based on the inflection point in 314. As described above, penetrometer data can be selected that appears before the inflection point and durometer data can be selected that appears after the inflection point. Any unselected data can be discarded in 316.

The computer system can generate the ripeness metric in 318. As described herein, the ripeness metric can be engineered using the penetrometer data 304 and the durometer data 306, both destructive measurements. More specifically, the ripeness metric can be engineered using the selected portions of each of the penetrometer data 304 and the durometer data 306. The ripeness metric can indicate how multiple different measurements are correlated during a ripening process of a particular food item.

The computer system can then generate the model in 320. The model can be trained to correlate the engineered ripeness metric with spectrometer data and other optional, non-destructive input data. In other words, the model can be trained to learn the mapping of spectra to the engineered ripeness metric. Thus, the ripeness metric generated in 318 can become a desired output for the trained machine learning model. The model can be generated and trained to receive non-destructive measurements as input and directly predict the engineered ripeness metric without destroying or otherwise invading a food item.

The model can then be outputted in 322. Once the model is outputted, the model can be used to non-destructively identify firmness or other ripeness levels of food items.

As described throughout this disclosure, models can be generated for each type of food item. For example, one or more models can be generated for avocados, apples, berries, etc. Different variations of models can also be generated based on specific characteristics of a same type of food item. For example, avocados from Mexico may have different ripening characteristics that are taken into account in a model versus avocados from California.

FIG. 3B is a flowchart of another process 350 for generating a model for determining food item ripeness. The process 350 can be performed by one or more of the computing systems described herein. For example, the process 350 can be performed by the computer system 190, the user device 192, and/or the system 100 (e.g., refer to the FIGS. 1A-C). For simplicity and exemplary purposes, the process 350 is described from the perspective of the computing system 190.

Referring to the process 350 in FIG. 3B, the computer system can receive training data in 352. The training data can include penetrometer data (354) and durometer data (356) that are obtained by performing physical testing on a food item using a penetrometer and/or a durometer, which can each measure the firmness of the food item. In some implementations, the skin of food items can be removed and then the training data can be obtained by performing physical testing on the skinless food items. Therefore, the physical testing can be performed directly on the flesh of the food items to generate accurate firmness measurements of the food items.

The penetrometer data 354 can further include force data (358) and depth data (360). The force data can indicate how much force (e.g., measured in gram-force, pound-force) is applied to the penetrometer as the penetrometer punctures a skin or outer layer of a food item. The depth data can indicate how far the penetrometer goes through or into the food item (e.g., measured in mm) when force is applied to the penetrometer. The force and depth data can indicate how much resistance if felt when the penetrometer enters and moves into the food item, which can be a corollary for firmness of the food item. Since the computer system is collecting a variety of data during training, the computer system can determine various engineered metrics that can be predicted by the models described herein.

Optionally, the computer system can normalize the data in 362. For example, the computer system can normalize the penetrometer data (354) and the durometer data (356). In some implementations, the computer system may normalize only some of the data, such as just the durometer data (356) or just the penetrometer data (354). The computer system may normalize the data in 362 for purposes of training a model with both penetrometer and durometer data. Refer to 372 for additional discussion.

The computer system can map the depth data to the force data in 364. For example, as shown in FIG. 3C, the computer system can generate a graph or otherwise plot the depth data (360) on an x axis and the force data (358) on a y axis of the graph.

Using the mapped data, the computer system can determine a slope (366). Refer to FIG. 3C. The slope can correlate to a firmness of the food item. The firmness can be a proxy for ripeness. The slope, as described further below, can be an engineered firmness metric. A model can be trained to predict the slope during runtime using non-destructively measured data, such as spectra data. The slope can be determined for a portion of the mapped penetrometer data, such as a portion of the mapped penetrometer data with a positive slope before a maximum force value. For example, such a slope can be determined as, for example, an average slope, a maximum slope, a median slope value, and/or other slope values for spans within the portion of the penetrometer data before the maximum force value is reached.

Optionally, the computer system can determine a max force of the mapped data (368). The max force can indicate yield stress and be a corollary to food item firmness. The max force can represent when the penetrometer punctures a food item's flesh, bends a little under the force applied to the penetrometer, and then breaks through the food item's flesh. A model can then be trained to predict the max force from spectra data as an engineered firmness metric for the food item. Refer to 372-376 for additional information about training the model. In some implementations, max force can be combined with the slope and/or area under the curve to generate the engineered firmness metric and train the model to predict the engineered firmness metric.

Optionally, the computer system can determine an area under curve (AUC) of the mapped data (370). The AUC can be a corollary to food item firmness. The computer system can determine the AUC for any range of received depth data 360, as described further in reference to FIG. 3C. The computer system can also determine the AUC after the max force. In some implementations, the computer system can determine an internal AUC, which can represent an area under the max force or a peak in the mapped data. A model can then be trained to predict the AUC from spectra data as the engineered firmness metric for the food item. Refer to 372-376 for additional discussion about training. In some implementations, the AUC can be combined with max force and/or the slope to generate the engineered firmness metric and train the model to predict that metric. Refer to FIG. 3C for additional discussion.

In 372, the computer system can map the durometer data and the determined slope to a single, comprehensive firmness metric using orthogonal regression and projection (366). This mapping can be performed in order to generate a comprehensive firmness curve that accounts for different types of food items, such as hard and soft food items. For example, the slope of the penetrometer data 354 can be used to accurately determine firmness of hard food items but may not be as sensitive for determining firmness of soft food items. Therefore, the computer system can use orthogonal regression and projection to perform the mapping and generate the comprehensive firmness curve that accounts for changes in firmness of various food item types (e.g., hard and soft food items).

Orthogonal regression and projection techniques can be used for examining a linear relationship between two continuous variables. Here, orthogonal regression is used to determine the relationship between normalized penetrometer slope and the normalized durometer values, as shown in FIG. 3D. Orthogonal projection is a form of linear transformation to project the normalized penetrometer slope and the normalized durometer values onto a single curve/line. The single curve/line therefore compresses data from 2 dimensions onto a single axis. In some implementations, the computer system can map one or more other metrics to build a single curve that compresses data from multiple dimensions onto a single axis. For example, the computer system can map the max force and durometer values onto a single curve representing the firmness metric. The computer system can also map the max force, slope, and durometer values onto a single curve. Sometimes, the computer system can map the AUC and durometer values onto a single curve. The computer system can also map the AUC, slope, and durometer values onto a single curve. In some implementations, the computer system can map the AUC, max force, and durometer values onto a single curve. Sometimes, the computer system can map the AUC, max force, slope, and durometer values onto a single curve. Thus, the computer system can project two dimensional, three dimensional, and four dimensional mappings to generate the comprehensive, engineered firmness metric.

The computer system can generate an engineered firmness metric based on the mapping in 374. As described herein, the firmness metric can be a proxy for the ripeness metric and can be engineered using the destructive measurements in 354-360. More specifically, the firmness metric can be engineered using the slope of the penetrometer data 354, as determined in 366. The slope of the penetrometer data 354 can be correlated to firmness of one or more types of food items, such as hard food items. The firmness metric can also be engineered using the mapping of the durometer data 356 to the slope of the penetrometer data 354, which can be correlated to firmness of one or more other types of food items, such as soft food items, and/or hard and soft food items.

The computer system can generate the training model in 376. The model can be trained to correlate the engineered firmness metric with spectrometer data and other optional, non-destructive input data. In other words, the model can be trained to learn the mapping of spectra to the engineered firmness metric. Thus, the firmness metric generated in 374 can become a desired output for the trained machine learning model. The model can be generated and trained to receive non-destructive measurements as input and directly predict the engineered firmness metric without destroying or otherwise invading a food item during runtime.

The computer system can then output the training model in 378. Once the model is outputted, the model can be used to non-destructively identify firmness or other ripeness levels of food items.

FIG. 3C illustrates firmness metrics 380 that can be determined using data collected for training a model to determine food item ripeness. As described above in reference to the process 350 in FIG. 3B, a computer system can train a model to predict an engineered firmness metric, which can be a proxy for food item ripeness (e.g., the engineered ripeness metric) described throughout this disclosure. As mentioned in 352 of the process 350 in FIG. 3B, the computer system can receive force and depth data as penetrometer data during model training. Mapping the force and depth data can yield the firmness metrics 380, which can include max force, area under the curve, and slope. The computer system can train a model to predict any of these firmness metrics 380 using spectra data or other non-destructive measurements during runtime.

The slope can be determined by finding the difference between 2 force endpoints within a depth range. The depth range can be, for example, 0.5-2mm. The depth range can also be 1.5-2mm. The depth range can be one or more other ranges, including but not limited to 1-3mm, 2.5-4.5mm, or 0.5-1.5mm. In some implementations, the depth range can be a longer or shorter range.

The AUC can be determined for any depth range so long as the force data is standardized and/or has been taken within the same depth range and/or at the same depth endpoints. For example, the AUC can be determined for a depth range of 0-8mm. The AUC can also be determined for a longer or shorter depth range. For example, the depth range can be 0-4mm, 0.5-2mm, 1.5-2.5mm, 1.5-4.5mm, 4-6mm, 5-6mm, or any other depth range.

As described in the process 350 in FIG. 3B, the slope of the penetrometer data can be used to accurately determine firmness of different types of food items. Graph 382 provides an understanding of why slope may be used to determine the firmness of the food items. Slope 386 corresponds to Young's modulus (Modulus of Elasticity), which indicates a stiffness of the material that is being punctured. The stiffness of the material can therefore correspond to a firmness of the food item. The greater the slope 386, the stiffer the material, which means the food item is firmer than a smaller slope (e.g., the food item has stiffer or harder flesh). Maximum force 384, on the other hand, is an indicator of yield strength, which represents a force required to break through the material. Although the maximum force 384 can still be used to determine firmness of the food item, the slope 386 can provide a more accurate quantification of the firmness of the food item.

FIG. 3D illustrates a firmness metric 390 that can be predicted by a model trained to determine food item ripeness. As described in FIGS. 3B-C, the firmness metric 390 shown herein can be a proxy for the engineered ripeness metric described throughout this disclosure. Ground truth graph 392 illustrates a mapping of normalized durometer data and normalized penetrometer data slope (or the slope) using orthogonal regression and projection. The graph 392 demonstrates consistent firmness behavior in two dimensional space of a particular type of food item, such as an avocado, as the food item ripens.

The durometer data and slope are mapped from two dimensional space and projected to a single, one dimensional line 394. This is beneficial since penetrometer data alone may not be the most accurate firmness indicator for soft food items while durometer data alone may not be the most accurate firmness indicator for hard food items. For example, food items that are at 0 to 0.5 on the x axis of the graph 392 may be very soft and mushy. Within this range of values, a penetrometer may not have sufficient sensitivity to determine firmness from only penetrometer data. Similarly, food items that are between 0.25 and 1 on the y axis of the graph 392 may be very firm. Within this range of values, a durometer may not have sufficient sensitivity to accurately determine firmness from only durometer data. Therefore, durometer and penetrometer data can be mapped and projected into the single line 394 using the techniques described herein in order to account for different types of food items (e.g., hard and soft food items).

The line 394 can represent firmness along a single bounded range of values between endpoints 396 and 398 of the line 394. The values of the endpoints 396 and 398 can vary. For example, the endpoint 396 can be a value of 0 and the endpoint 398 can be a value of 100, where 100 represents highest level of firmness and 0 represents lowest level of firmness. As another example, the endpoint 396 can have a value of 0 and the endpoint 398 can have a value of 5. As another example, the endpoint 396 can have a value of -1 and the endpoint 398 can have a value of 1. One or more other ranges of values can be defined by the endpoints 396 and 398.

The slope, represented by the Y axis of the graph 392, can be the engineered firmness metric 390 that the model is trained to predict during runtime using spectra data or other non-destructive measurements. As described above, the firmness metric 390 can also be max force, AUC, or any combination thereof. The firmness metric 390 can be used for predicting firmness of various types of food items, including but not limited to hard and soft fruits.

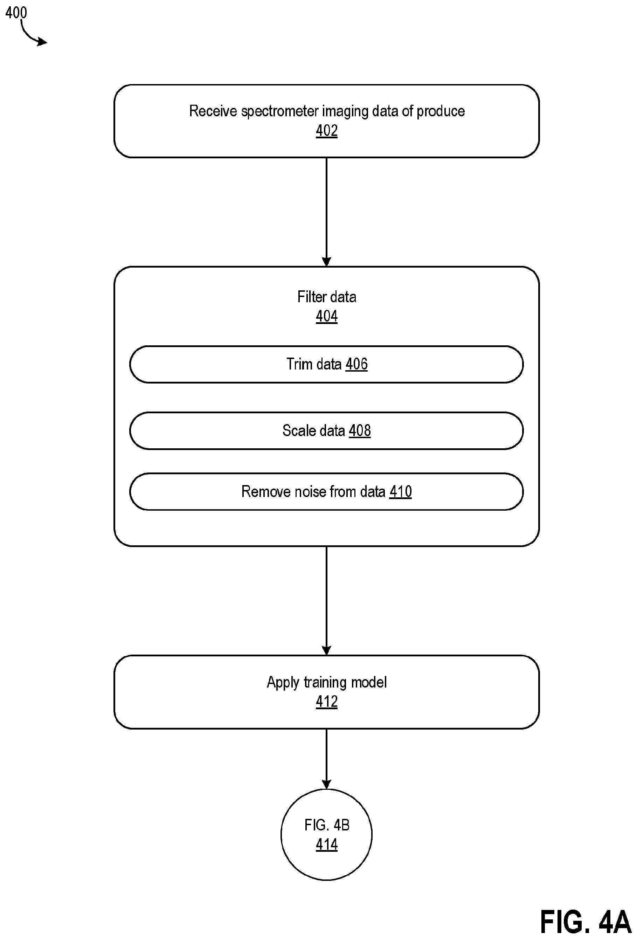

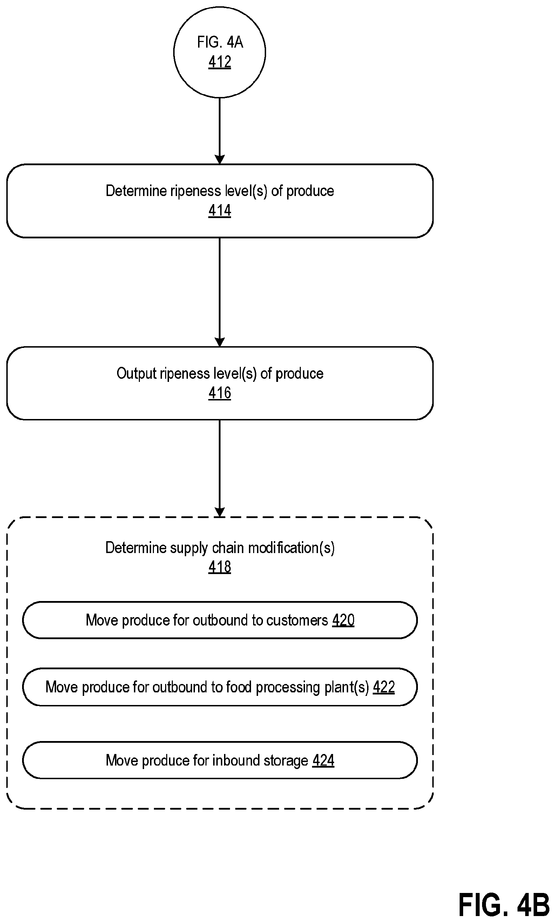

FIGS. 4A-B is a flowchart of a process 400 for determining food item ripeness using the model in real-time. The process 400 can be performed by one or more of the computing systems described herein. For example, the process 400 can be performed by the computer system 190, the user device 192, and/or the system 100 (e.g., refer to the FIGS. 1A-C). For simplicity and exemplary purposes, the process 400 is described from the perspective of the computing system 190.

Referring to the process 400 in both FIGS. 4A-B, the computer system can receive spectrometer imaging data of produce in 402. As described herein, in a facility (e.g., storage facility, warehouse), produce can be moved to different locations along conveyor belts. Spectral imaging device(s) can be positioned along the conveyor belts and configured to capture imaging data of the produce. The imaging data can include wavelength information that can be used with the model to identify a ripeness, firmness, and/or suitability for consumption level for that produce. In some implementations, the process 400 can be performed to identify the ripeness level for a batch of produce. In some implementations, the process 400 can be performed to identify the ripeness level for a particular produce in the batch. In yet other implementations, a group ripeness level can be extrapolated from the ripeness level of the particular produce and/or ripeness levels of other individual produce in the batch.

The computer system can filter the spectrometer imaging data in 404. The received spectrometer data can be raw input. Data processing techniques can be used to optimize this raw input data to improve ripeness identification once the model is applied. Filtering the data can include trimming the data in 406, scaling the data in 408, and/or removing noise from the data in 410 (e.g., refer to FIG. 7). A preferred input for use with the model can be amplitude of light in a frequency band. The amplitude of light can be more clearly defined in the received data by filtering the data in 404.

The model can be applied in 412. In other words, the model can be applied to the filtered spectrometer data. By applying the model, the computer system can determine ripeness level(s) of produce in 414. As described herein, the model can be trained to receive the filtered spectrometer data and map it to a ripeness metric that was engineered by the computer system (e.g., refer to FIGS. 1A, 3).Thus, the computer system can predict the ripeness level of the produce using the non-destructive spectrometer data.

The computer system can then output the ripeness level(s) of the produce in 416. As described herein, the ripeness level(s) can be outputted at the user device 192. The output can be a numeric value indicating the ripeness level(s). The output can also be a graphical depiction of the ripeness level(s). The output can be one or more other preferred forms of output to display the ripeness level(s) of the produce.

The computer system can optionally determine supply chain modifications in 418. Example modifications include moving the produce for outbound to customers in 420, moving produce for outbound to food processing plant(s) in 422, and/or moving produce for inbound storage in 424. In some implementations, it can be preferred to move the produce for outbound to customers (420) where the produce is at a preferred ripeness level. Therefore, the produce is ready for immediate consumption. It can be preferred to move the produce for outbound to food processing plant(s) (422) if the produce is overripe. The produce may be less likely to be purchased by consumers so it can be more efficient and cost-effective to use that produce in processed food. It can be preferred to move the produce for inbound storage (424) where the produce has not yet reached the preferred ripeness level. One or more other supply chain modifications can be determined and/or implemented in 418.

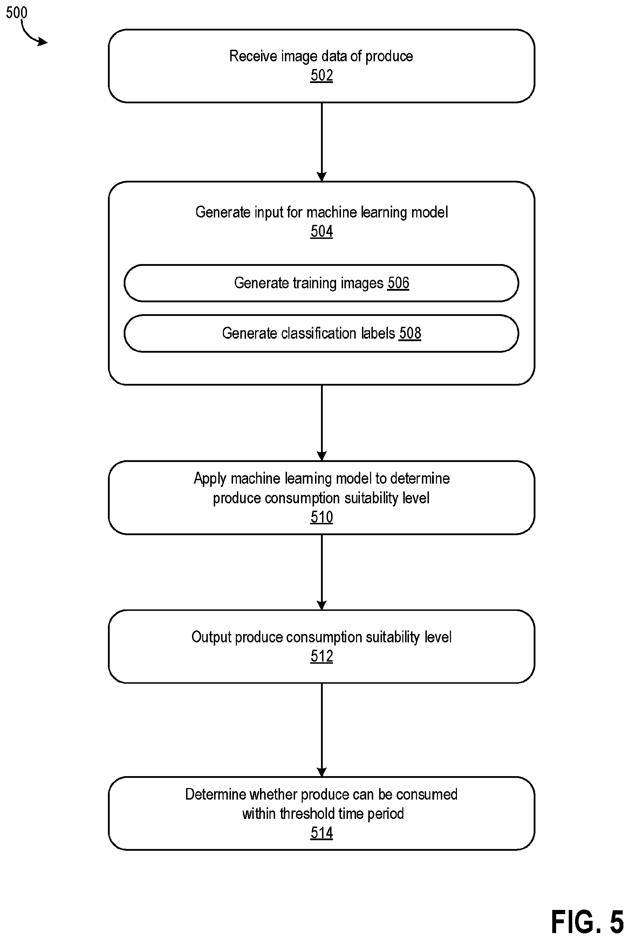

FIG. 5 is another flowchart of a process 500 for non-destructively determining whether the food item is ready for consumption using the model described herein. The process 500 can be performed by one or more of the computing systems described herein. For example, the process 500 can be performed by the computer system 190, the user device 192, and/or the system 100 (e.g., refer to the FIGS. 1A-C). For simplicity and exemplary purposes, the process 500 is described as being performed by a system such as the system 100.

Referring to the process 500, the system can receive image data of produce in 502. In some implementations, this can include obtaining a hyperspectral image of a food item. The image can be generated by one or more hyperspectral image sensors. As described herein, the image data can also be generated by one or more spectral imaging devices (e.g., NIR device). In some implementations, the obtained image data can be generated using a spectral imaging device using light having a wavelength of 534 nm to 942 nm. In other implementations, the spectral imaging device can use light having a wavelength of 690 nm to 912 nm and/or 672 nm to 948 nm.

The system can generate input for a machine learning model in 504. In some implementations, 504 can include encoding the data obtained in 502 into a vector that has one or more fields that organize a numerical representation of the image data from 502. Generating the input can include generating training images in 506 and generating classification labels in 508. The training images can include data pertaining to the produce, such as wavelength amplitudes, that can be used to measure consumption suitability of the produce. The classification labels can describe produce classification based on the training images. The classification labels can be based on first produce suitability data determined using non-destructive measurement(s) of the produce (e.g., spectrometer data) and second produce suitability data determined using destructive measurement(s) (e.g., durometer, penetrometer data) (e.g., refer to model generation in FIGS. 1A, 3). In some implementations, the labels that describes the produce classification for the produce depicted by the training images can include a produce score that can be determined by transforming (a) first produce suitability data obtained using a non-destructive measurement of the produce and (b) second produce suitability data obtained using a destructive measurement of the produce into a single value.

In 510, the computer system can apply the machine learning model to determine a produce consumption suitability level. The machine learning model can be trained to generate output indicative of a level of consumption suitability of a food item using a plurality of training data items. The training data items can include (i) the training images of the produce and (ii) the labels that describe the produce classifications for the produce depicted by the training images. By applying the machine learning model, the system can determine the produce consumption suitability level in a non-destructive manner during real-time application and use. The system can process, using the machine learning model, the generated input in order to generate output data indicative of the level of consumption suitability of the produce. For example, the machine learning model can process the generated input data through each layer of the trained machine learning model to generate the output data. The machine learning model can include multiple layers and/or one or more neural networks. Moreover, in some implementations, the produce consumption suitability level can include a probability that the produce represented by the obtained image has one or more properties that make the produce suitable for consumption.

The system can output the produce consumption suitability level in 512. This output data can indicate the level of consumption suitability for the produce that is depicted in the obtained image data. The output data can be generated by the trained machine learning model based on the trained machine learning model processing the obtained image.

In 514, the system can determine whether the produce can be consumed within a threshold time period. The obtained output data can be used to make this determination. Although not depicted in FIG. 5, the determination in 514 can be used to make one or more supply chain modifications. For example, if the produce can be consumed within the threshold time period, then the produce can be directed for outbound movement to end-consumers (e.g., grocery stores) for immediate purchase and consumption. As another example, if the produce cannot be consumed within the threshold time period, then the produce can be directed for outbound movement to food processing plants. As yet another example, if the produce can be consumed within the threshold time period, then the system can determine that the produce is in fact suitable for consumption (e.g., a preferred ripeness and/or firmness). If the produce cannot be consumed within the threshold time period, then the system can determine that the produce is not suitable for consumption (e.g., is overripe and/or too soft).

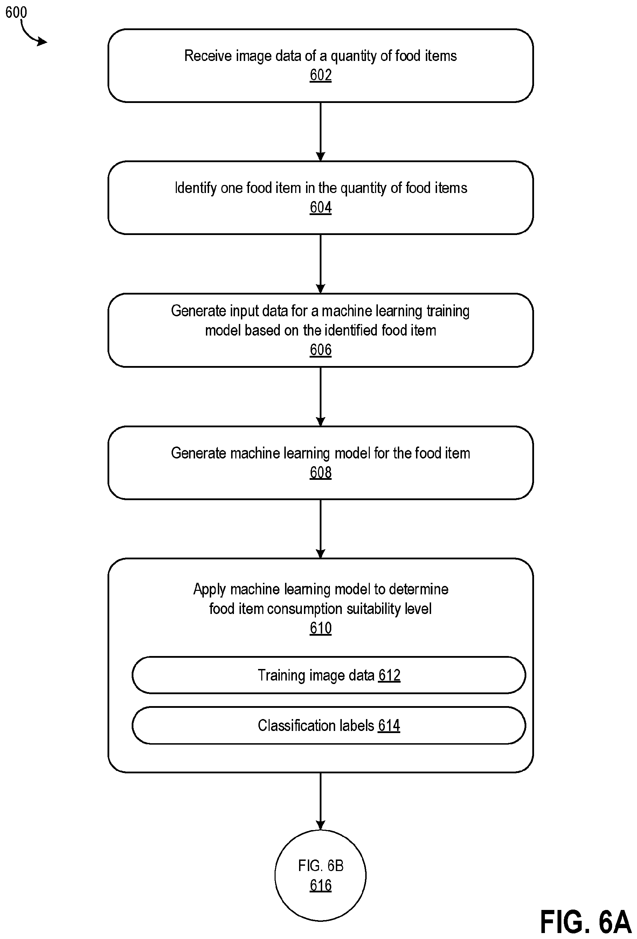

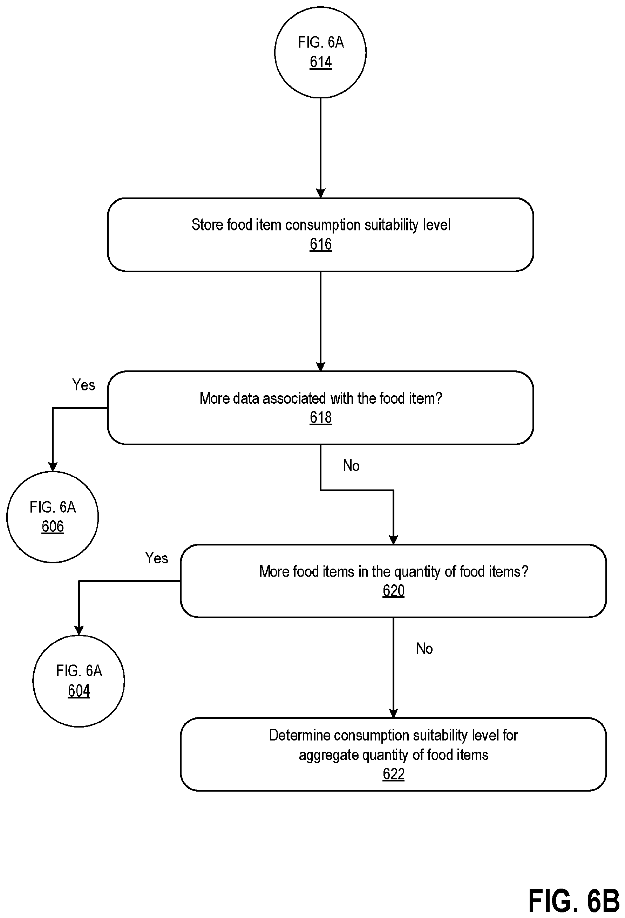

FIGS. 6A-B is a flowchart of a process 600 for non-destructively determining whether a batch of food items are ready for consumption using the model. The process 600 can be performed by one or more of the computing systems described herein. For example, the process 600 can be performed by the computer system 190, the user device 192, and/or the system 100 (e.g., refer to the FIGS. 1A-C). For simplicity and exemplary purposes, the process 600 is described as being performed by a system such as the system 100.

Referring to the process 600 in both FIGS. 6A-B, the system can receive image data of a quantity of food items in 602. In some implementations, for example, the system can use hyperspectral image sensors to generate a hyperspectral image of a plurality of food items, such as avocados. The generated image can be stored in a memory device. One or more other processing engines can obtain the generated image from the image sensors, cameras, and/or from the memory. Thus, obtaining the image data in 602 can include generating an image by one or more image sensors (e.g., spectral imaging device, NIR device, hyperspectral imaging device), receiving the generated image from the one or more images sensors, and/or obtaining the image from a memory device.

The system can identify one food item in the quantity of food items in 604. For example, the system can obtain first data corresponding to a first portion of the obtained image. The first portion of the obtained image can represent a particular food item of the one or more food items in the quantity of food items. In some implementations, for example, the system can obtain the portion of the obtained image that corresponds to a single avocado from the obtained image that depicts the plurality of avocados. The portion of the obtained image can be identified and obtained using object recognition techniques as described herein.

Moreover, in 606, the system can generate input data for a machine learning model based on the identified food item. For example, the system can obtain second data corresponding to a portion of the first data. The input data can be generated for each pixel of the obtained portion of the first image that corresponds to the particular food item. In some implementations, for example, the system can obtain data describing a spectrum of light waves associated with a particular pixel of an image of a single food item, such as an avocado. In some implementations, for example, this generated input data can include a vector that corresponds to the obtained data for the identified food item. In such implementations, generating of the input data can include encoding obtained data describing a spectrum of light waves associated with a particular pixel of the image of the single food item into a vector. The encoded vector can include a numerical representation of the second data described herein.

As described throughout this disclosure, the computer system can generate a machine learning model for the food item in 608. In some implementations, the machine learning model can already be generated as described herein (e.g., refer to FIGS. 1A, 3). In some implementations, the machine learning model can be generated in real-time at a similar time as the machine learning model is used to non-destructively determine consumption suitability of the food item(s).

In 610, the system can apply the machine learning model to determine a food item consumption suitability level. Training image data 612 and classification labels 614 can be used in applying the machine learning model. Thus, the system can, using the machine learning model, process the generated input in order to generate output data indicative of the level of consumption suitability of the food item using a plurality of training data items. As described above in reference to FIG. 5, each training data item of the plurality of training data items can include (i) a training image of a food item and (ii) a label that describes food item classification for the food item depicted by the training image. The food item classification can be based on (a) first food item suitability data obtained using a non-destructive measurement of the food item and (b) second food item suitability data obtained using a destructive measurement of the food item. For example, the machine learning model can process the generated input data through each layer of the trained machine learning model to generate output data.

In 616, the system can store the food item consumption suitability level (e.g., the output data). This can include, for example, obtaining the output data generated by the machine learning model and storing the obtained output data in one or more memory devices. The output data generated by the machine learning model can include data indicating a suitability for consumption for the particular portion of the obtained first data represented by the second data obtained at 606. In some implementations, the output data can include one or more values representing a predicted destructive measurement of the food item that is non-destructively inferred by the machine learning model. In some implementations, the one or more values can be a predicted non-destructive measurement and a predicted destructive measurement. Alternatively, the one or more values can be a single value such as a slope of a predicted non-destructive measurement and a predicted destructive measurement.

The system can then determine whether more data is associated with the food item in 618. In other words, the system can determine whether there is another portion of the obtained first data that can be analyzed. For example, in some implementations, the system can determine whether there is another pixel of the first portion of the obtained image that can be analyzed. If there is another pixel, then the system can return to 606 and repeat 606-618 for each remaining pixel of the first portion of the obtained image.

On the other hand, if there is no more data associated with the food item (e.g., no additional pixels of the first portion of the obtained image that can be analyzed), then the system can determine whether more food items exist in the quantity of food items in 620. The system can determine whether another food item is depicted in the image obtained in 602. If there is another food item, then the system can return to 604 and repeat 604-618 for each remaining food item in the quantity of food items.

Alternatively, if there are no more food items in the quantity of food items, the system can determine a consumption suitability level for an aggregate quantity of food items in 622. This determination can be based on the stored output data in memory. Analyzing the output data stored in the memory device can include accessing an aggregate representation of output data stored in the memory device for each pixel of each image of each food item depicted by the image data from 602 and analyzing an aggregate representation of output data, as described herein. Thus, the system can determine a consumption suitability level for each of the food items in the quantity of food items as well as an aggregate consumption suitability level for the quantity of food items.

In some implementations, as described in reference to FIGS. 5-6, the system can determine consumption suitability for a food item on a pixel-by-pixel basis. For example, the system can obtain an image of the food item and for each pixel of the image of the food item, the system can generate input data for the pixel, provide the generated input data as an input to a machine learning model that has been trained to generate output data indicative of a level of consumption suitability of a food item using a plurality of training data items, obtain output data generated by the trained machine learning model based on the trained machine learning model processing the obtained image, storing the obtained output data in a memory device, and determine, based on the stored output data generated for each pixel of the image, whether the food item depicted by the obtained image is suitable for consumption. Moreover, the system can determine whether the food item is suitable for consumption based on generating an aggregated value based on the stored output data for each pixel of the image. In some implementations, the system can determine that the aggregated value satisfies a predetermined threshold, which can indicate that the food item is suitable for consumption. In some implementations, the system can determine that the aggregated value does not satisfy a predetermined threshold, which can indicate that the food item is not suitable for consumption.

FIG. 7 is a graphical depiction of determining food item ripeness using the model described herein. FIG. 7 is an example experiment in which firmness is determined for avocados. Raw spectral data 702 can be received. As described herein, this data can be NIR data. The data can be processed 704. Processing the data can include trimming and scaling the spectral data. A Savitzky-Golay 2^{nd} derivative filter can also be applied to filter the spectral data. In an example experiment, raw NIR spectral data for each of ~2000 avocados can be trimmed, scaled, and filtered to eliminate noise from the data and make differences in the spectra more apparent. Thus, a filtered spectral data graph 706 can be outputted.

A model can be applied to the filtered spectral data 708. In the example experiment, a Partial Least Squares (PLS) model can be used to demonstrate a maximum variance in an Extended Shore Projection line (e.g., refer to FIG. 3). The Extended Shore Projection line can provide a single reference value that can be used for model development from NIR data. Using the model, a firmness level (e.g., ripeness level, consumption suitability level) can be predicted 712.

The predicted firmness level can be outputted in a firmness output graph 714. The graph 714 can indicate firmness, ripeness, or consumption suitability of the produce. As shown in the example graph 714, a median of 3 durometer measurements are plotted on an x-axis and a mean of 2 penetrometer measurements are plotted on a y-axis for nearly 4,000 avocados at different ripeness stages. These avocados also come from different origins, including Mexico, Peru, and California. As depicted, the penetrometer is sensitive to firmness changes in unripe (hard) avocados, but is insensitive to changes in firmness of soft avocados, especially below 11b (70 shore). Alternatively, the durometer is sensitive to firmness changes in soft avocados (<70 shore) but insensitive to changes in firmness of hard (unripe) avocados. A point at which the penetrometer is insensitive to soft avocados and the durometer is insensitive to hard avocados can be represented by an inflection point, as described herein (e.g., refer to FIG. 3).

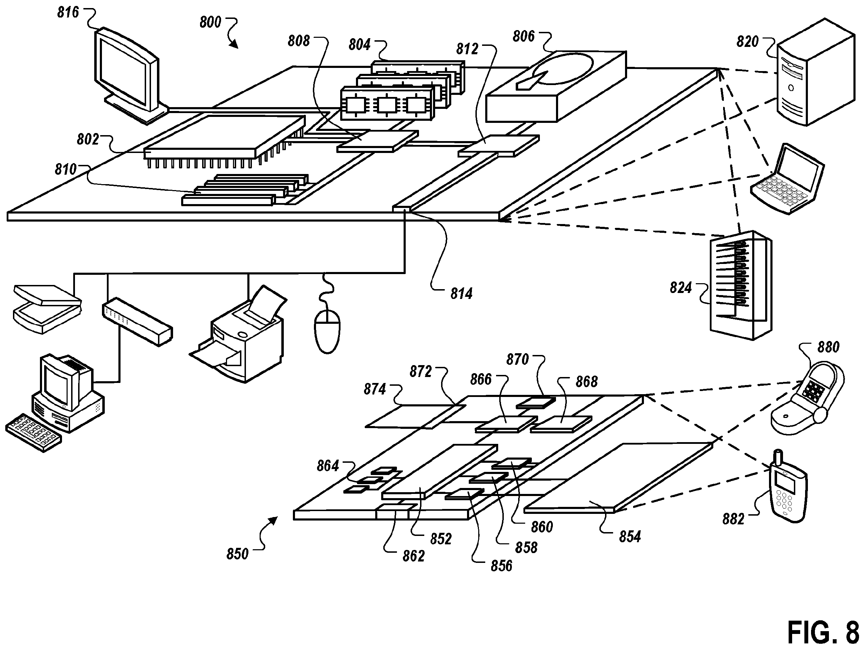

FIG. 8 is a block diagram of system components that can be used to implement a system for non-destructively determining whether a food item is suitable for consumption based on output data generated by a trained machine learning model. The computing device 800 is intended to represent various forms of digital computers, such as laptops, desktops, workstations, personal digital assistants, servers, blade servers, mainframes, and other appropriate computers. The mobile computing device is intended to represent various forms of mobile devices, such as personal digital assistants, cellular telephones, smart-phones, and other similar computing devices. The components shown here, their connections and relationships, and their functions, are meant to be exemplary only, and are not meant to limit implementations of the inventions described and/or claimed in this document.

The computing device 800 includes a processor 802, a memory 804, a storage device 806, a high-speed interface 808 connecting to the memory 804 and multiple high-speed expansion ports 810, and a low-speed interface 812 connecting to a low-speed expansion port 814 and the storage device 806. Each of the processor 802, the memory 804, the storage device 806, the high-speed interface 808, the high-speed expansion ports 810, and the low-speed interface 812, are interconnected using various busses, and can be mounted on a common motherboard or in other manners as appropriate. The processor 802 can process instructions for execution within the computing device 800, including instructions stored in the memory 804 or on the storage device 806 to display graphical information for a GUI on an external input/output device, such as a display 816 coupled to the high-speed interface 808. In other implementations, multiple processors and/or multiple buses can be used, as appropriate, along with multiple memories and types of memory. Also, multiple computing devices can be connected, with each device providing portions of the necessary operations (e.g., as a server bank, a group of blade servers, or a multi-processor system).

The memory 804 stores information within the computing device 800. In some implementations, the memory 804 is a volatile memory unit or units. In some implementations, the memory 804 is a non-volatile memory unit or units. The memory 804 can also be another form of computer-readable medium, such as a magnetic or optical disk.

The storage device 806 is capable of providing mass storage for the computing device 800. In some implementations, the storage device 806 can be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product can also contain instructions that, when executed, perform one or more methods, such as those described above. The computer program product can also be tangibly embodied in a computer- or machine-readable medium, such as the memory 804, the storage device 806, or memory on the processor 802.

The high-speed interface 808 manages bandwidth-intensive operations for the computing device 800, while the low-speed interface 812 manages lower bandwidth-intensive operations. Such allocation of functions is exemplary only. In some implementations, the high-speed interface 808 is coupled to the memory 804, the display 816 (e.g., through a graphics processor or accelerator), and to the high-speed expansion ports 810, which can accept various expansion cards (not shown). In the implementation, the low-speed interface 812 is coupled to the storage device 806 and the low-speed expansion port 814. The low-speed expansion port 814, which can include various communication ports (e.g., USB, Bluetooth^{®}, Ethernet, wireless Ethernet) can be coupled to one or more input/output devices, such as a keyboard, a pointing device, a scanner, or a networking device such as a switch or router, e.g., through a network adapter.

The computing device 800 can be implemented in a number of different forms, as shown in the figure. For example, it can be implemented as a standard server 820, or multiple times in a group of such servers. In addition, it can be implemented in a personal computer such as a laptop computer 822. It can also be implemented as part of a rack server system 824. Alternatively, components from the computing device 800 can be combined with other components in a mobile device (not shown), such as a mobile computing device 850. Each of such devices can contain one or more of the computing device 800 and the mobile computing device 850, and an entire system can be made up of multiple computing devices communicating with each other.

The mobile computing device 850 includes a processor 852, a memory 864, an input/output device such as a display 954, a communication interface 866, and a transceiver 868, among other components. The mobile computing device 850 can also be provided with a storage device, such as a micro-drive or other device, to provide additional storage. Each of the processor 852, the memory 864, the display 854, the communication interface 866, and the transceiver 868, are interconnected using various buses, and several of the components can be mounted on a common motherboard or in other manners as appropriate.

The processor 852 can execute instructions within the mobile computing device 850, including instructions stored in the memory 864. The processor 852 can be implemented as a chipset of chips that include separate and multiple analog and digital processors. The processor 852 can provide, for example, for coordination of the other components of the mobile computing device 850, such as control of user interfaces, applications run by the mobile computing device 850, and wireless communication by the mobile computing device 850.

The processor 852 can communicate with a user through a control interface 858 and a display interface 856 coupled to the display 854. The display 854 can be, for example, a TFT (Thin-Film-Transistor Liquid Crystal Display) display or an OLED (Organic Light Emitting Diode) display, or other appropriate display technology. The display interface 856 can comprise appropriate circuitry for driving the display 854 to present graphical and other information to a user. The control interface 858 can receive commands from a user and convert them for submission to the processor 852. In addition, an external interface 862 can provide communication with the processor 852, so as to enable near area communication of the mobile computing device 850 with other devices. The external interface 862 can provide, for example, for wired communication in some implementations, or for wireless communication in other implementations, and multiple interfaces can also be used.

The memory 864 stores information within the mobile computing device 850. The memory 864 can be implemented as one or more of a computer-readable medium or media, a volatile memory unit or units, or a non-volatile memory unit or units. An expansion memory 874 can also be provided and connected to the mobile computing device 850 through an expansion interface 872, which can include, for example, a SIMM (Single In Line Memory Module) card interface. The expansion memory 874 can provide extra storage space for the mobile computing device 850, or can also store applications or other information for the mobile computing device 850. Specifically, the expansion memory 874 can include instructions to carry out or supplement the processes described above, and can include secure information also. Thus, for example, the expansion memory 874 can be provide as a security module for the mobile computing device 850, and can be programmed with instructions that permit secure use of the mobile computing device 850. In addition, secure applications can be provided via the SIMM cards, along with additional information, such as placing identifying information on the SIMM card in a non-hackable manner.

The memory can include, for example, flash memory and/or NVRAM memory (non-volatile random access memory), as discussed below. In some implementations, a computer program product is tangibly embodied in an information carrier. The computer program product contains instructions that, when executed, perform one or more methods, such as those described above. The computer program product can be a computer- or machine-readable medium, such as the memory 864, the expansion memory 874, or memory on the processor 852. In some implementations, the computer program product can be received in a propagated signal, for example, over the transceiver 868 or the external interface 862.

The mobile computing device 850 can communicate wirelessly through the communication interface 866, which can include digital signal processing circuitry where necessary. The communication interface 866 can provide for communications under various modes or protocols, such as GSM voice calls (Global System for Mobile communications), SMS (Short Message Service), EMS (Enhanced Messaging Service), or MMS messaging (Multimedia Messaging Service), CDMA (code division multiple access), TDMA (time division multiple access), PDC (Personal Digital Cellular), WCDMA (Wideband Code Division Multiple Access), CDMA2000, or GPRS (General Packet Radio Service), among others. Such communication can occur, for example, through the transceiver 868 using a radio-frequency. In addition, short-range communication can occur, such as using a Bluetooth^{®}, Wi-Fi^{™}, or other such transceiver (not shown). In addition, a GPS (Global Positioning System) receiver module 870 can provide additional navigation- and location-related wireless data to the mobile computing device 850, which can be used as appropriate by applications running on the mobile computing device 850.

The mobile computing device 850 can also communicate audibly using an audio codec 860, which can receive spoken information from a user and convert it to usable digital information. The audio codec 860 can likewise generate audible sound for a user, such as through a speaker, e.g., in a handset of the mobile computing device 850. Such sound can include sound from voice telephone calls, can include recorded sound (e.g., voice messages, music files, etc.) and can also include sound generated by applications operating on the mobile computing device 850.

The mobile computing device 850 can be implemented in a number of different forms, as shown in the figure. For example, it can be implemented as a cellular telephone 880. It can also be implemented as part of a smart-phone 882, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms machine-readable medium and computer-readable medium refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term machine-readable signal refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing system that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network (LAN), a wide area network (WAN), and the Internet.

The computing system can include clients and servers. A client and server are generally remote from each other and typically interact through a communication network. The relationship of client and server arises by virtue of computer programs running on the respective computers and having a client-server relationship to each other.

While this specification contains many specific implementation details, these should not be construed as limitations on the scope of the claims, but rather as descriptions of features that may be specific to particular embodiments of particular disclosed technologies. Certain features that are described in this specification in the context of separate embodiments can, within the scope defined by the appended claims, also be implemented in combination in a single embodiment in part or in whole. Conversely, various features that are described in the context of a single embodiment can, within the scope defined by the appended claims, also be implemented in multiple embodiments separately or in any suitable subcombination. Similarly, while operations may be described in a particular order, this should not be understood as requiring that such operations be performed in the particular order or in sequential order, or that all operations be performed, to achieve desirable results. Particular embodiments of the subject matter have been described. Other embodiments are within the scope of the following claims.

## Claims

1. A method for generating a trained model to determine a ripeness metric of a food item (102), the method comprising:
receiving (300), by a computing system (190), (i) penetrometer data (114) from one or more penetrometers and (ii) durometer data (116) from one or more durometers for a plurality of test food items (102) of a same food type; **characterized in that** the method further comprises:
selecting (308), by the computing system (190), portions of the penetrometer data (114) and the durometer data (116);
determining (318), by the computing system (190), a ripeness metric for food items (102) of the same food type based on the selected portions of the penetrometer data (114) and the durometer data (116); and
generating (320), by the computing system (190), a machine learning trained model (130) based on the ripeness metric, wherein the machine learning trained model (130) correlates destructive measurements provided by the selected portions of the penetrometer data (114) and the selected portions of the durometer data (116) with non-destructive measurements provided by spectral data to model the ripeness metric for the food items (102) of the same food type.

2. The method of claim 1, wherein selecting (308) portions of the penetrometer data and the durometer data comprises:
plotting the penetrometer data and the durometer data;
identifying an inflection point in the plotted penetrometer data and the plotted durometer data;
selecting portions of the penetrometer data and the durometer data based on the inflection point; and
discarding unselected portions of the penetrometer data and the durometer data.

3. The method of claim 2, wherein selecting portions of the penetrometer and durometer data based on the inflection point comprises selecting portions of the penetrometer data before the inflection point and selecting portions of the durometer data after the inflection point.

4. The method of claim 3, further comprising discarding portions of the durometer data before the inflection point and discarding portions of the penetrometer data after the inflection point.

5. The method of claim 2, wherein generating (320) the machine learning trained model comprises (i) correlating the selected portions of the penetrometer data before the inflection point with one or more wavelengths of spectral data that correspond to the plurality of test food items that are hard and (ii) correlating the selected portions of the durometer data after the inflection point with one or more wavelengths of spectral data that correspond to the test food items that are soft.

6. The method of claim 1, wherein the machine learning trained model further correlates destructive measurements provided by the selected portions of the penetrometer data and the selected portions of the durometer data with at least one of (i) a place of origin, (ii) a storage temperature, and (iii) historic ripening information associated with the food items of the same food type.

7. The method of claim 1, further comprising:
receiving, by a computing system and from a spectral imaging device, spectral data of a food item;
filtering, by the computing system, the spectral data;
determining, by the computing system and based on applying the machine learning trained model to the filtered spectral data, a ripeness level of the food item, wherein the machine learning trained model was trained using (i) one or more destructive measurements of other food items and (ii) spectral data for the other food items, wherein the other food items are of a same food type as the food item, wherein the ripeness level of the food item is determined without taking destructive measurements of the food item; and
transmitting, by the computing system to a user computing device, the ripeness level of the food item for display at the user computing device.

8. The method of claim 7, wherein the spectral data includes one or more non-destructive measurements of the food item.

9. The method of claim 7, wherein the machine learning trained model includes one or more layers, wherein each of the layers includes (i) training images of the other food items and (ii) labels that indicate food item classifications for each of the other food items depicted by the training images.

10. The method of claim 7, wherein filtering the spectral data includes:
trimming the spectral data;
scaling the spectral data; and applying a Savitzky-Golay 2^{nd} derivative filter to the spectral data to reduce noise.

11. The method of claim 7, further comprising determining that the food item is suitable for consumption based on the ripeness level of the food item exceeding a threshold value.

12. The method of claim 7, further comprising determining that the food item is not suitable for consumption based on the ripeness level of the food item being less than a threshold value.

13. The method of claim 7, the method comprising:
receiving, by a computing system, the ripeness level of the food item of a food item type, wherein the ripeness level is determined using a non-destructive measurement of the food item and a trained model for the food item type, wherein the trained model was trained using one or more destructive measurements of other food items of the food item type;
identifying, by the computing system, supply chain information for the food item that includes a preexisting supply chain schedule and destination for the food item;
determining, by the computing system, whether to modify the supply chain information for the food item based on the received ripeness level;
in response to a determination to modify the supply chain information, generating, by the computing system, modified supply chain information based on the received ripeness level, wherein the modified supply chain information includes one or more of a modified supply chain schedule and modified destination for the food item; and
transmitting, by the computing system, the modified supply chain information to one or more supply chain actors to implement the modified supply chain information.

14. The method of claim 13, further comprising:
determining, by the computing system and based on the received ripeness level exceeding a threshold value, that the food item is suitable for consumption by end-consumers; and
determining, by the computing system and based on the received ripeness level being less than the threshold value, that the food item is not suitable for consumption by the end-consumers.

15. A system (100) for determining ripeness levels for food items (102) using non-contact assessments of the food items (102), the system (100) comprising:
one or more penetrometers configured to measure penetrometer data (114) for a plurality of test food items (102) of a same food type;
one or more durometers configured to measure durometer data (116) for the plurality of test food items (102) of the same food type;
one or more spectral imaging devices (194) configured to measure spectral data for food items (102) of the same food type; and
at least one computing system (190, 106) configured to perform the method of any one of claims 1 to 12.

## Patentansprüche

1. Verfahren zur Erzeugung eines trainierten Modells zur Bestimmung eines Reifemaßes für ein Lebensmittel (102),
wobei das Verfahren umfasst:
das Empfangen (300) von (i) Penetrometerdaten (114) von einem oder mehreren Penetrometern und (ii) Durometerdaten (116) von einem oder mehreren Durometern für eine Vielzahl von Testlebensmitteln (102) desselben Lebensmitteltyps durch ein Computersystem (190),
**dadurch gekennzeichnet, dass** das Verfahren weiterhin umfasst:
das Auswählen (308) von Teilen der Penetrometerdaten (114) und der Durometerdaten (116) durch das Computersystem (190);
das Bestimmen (318) eines Reifemaßes für Lebensmittel (102) desselben Lebensmitteltyps durch das Computersystem (190) anhand der ausgewählten Teile der Penetrometerdaten (114) und der Durometerdaten (116); und
das Erzeugen (320) eines durch Maschinenlernen trainierten Modells (130) durch das Computersystem (190) anhand des Reifemaßes, wobei das durch Maschinenlernen trainierte Modell (130) zerstörende Messungen, die durch die ausgewählten Teile der Penetrometerdaten (114) und die ausgewählten Teile der Durometerdaten (116) bereitgestellt werden, mit zerstörungsfreien Messungen korreliert, die durch Spektraldaten bereitgestellt werden, um das Reifemaß für die Lebensmittel (102) desselben Lebensmitteltyps zu modellieren.

2. Verfahren gemäß Anspruch 1, wobei das Auswählen (308) von Teilen der Penetrometerdaten und der Durometerdaten Folgendes umfasst:
das Auftragen der Penetrometerdaten und der Durometerdaten;
das Identifizieren eines Wendepunkts in den aufgetragenen Penetrometerdaten und den aufgetragenen Durometerdaten;
das Auswählen von Teilen der Penetrometerdaten und der Durometerdaten auf der Basis des Wendepunkts; und
das Verwerfen nicht ausgewählter Teile der Penetrometerdaten und der Durometerdaten.

3. Verfahren gemäß Anspruch 2, wobei das Auswählen von Teilen der Penetrometer- und Durometerdaten auf der Basis des Wendepunkts das Auswählen von Teilen der Penetrometerdaten vor dem Wendepunkt und das Auswählen von Teilen der Durometerdaten nach dem Wendepunkt umfasst.

4. Verfahren gemäß Anspruch 3, weiterhin umfassend das Verwerfen von Teilen der Durometerdaten vor dem Wendepunkt und das Verwerfen von Teilen der Penetrometerdaten nach dem Wendepunkt.

5. Verfahren gemäß Anspruch 2, wobei das Erzeugen (320) des durch Maschinenlernen trainierten Modells Folgendes umfasst: (i) das Korrelieren der ausgewählten Teile der Penetrometerdaten vor dem Wendepunkt mit einer oder mehreren Wellenlängen von Spektraldaten, die der Vielzahl von Testlebensmitteln entsprechen, welche hart sind, und (ii) das Korrelieren der ausgewählten Teile der Durometerdaten nach dem Wendepunkt mit einer oder mehreren Wellenlängen von Spektraldaten, die den Testlebensmitteln entsprechen, welche weich sind.

6. Verfahren gemäß Anspruch 1, wobei das durch Maschinenlernen trainierte Modell weiterhin zerstörende Messungen, die durch die ausgewählten Teile der Penetrometerdaten und die ausgewählten Teile der Durometerdaten bereitgestellt werden, mit (i) einem Herkunftsort, (ii) einer Lagerungstemperatur und/oder (iii) historischen Reifungsinformationen, die mit den Lebensmitteln desselben Lebensmitteltyps verbunden sind, korreliert.

7. Verfahren gemäß Anspruch 1, weiterhin umfassend:
das Empfangen von Spektraldaten eines Lebensmittels von einem Spektralbildgebungsgerät durch ein Computersystem,
das Filtern der Spektraldaten durch das Computersystem;
das Bestimmen eines Reifegrads des Lebensmittels durch das Computersystem aufgrund der Anwendung des durch Maschinenlernen trainierten Modells auf die gefilterten Spektraldaten, wobei das durch Maschinenlernen trainierte Modell mit Hilfe von (i) einer oder mehreren zerstörenden Messungen von anderen Lebensmitteln und (ii) Spektraldaten für die anderen Lebensmittel trainiert wurde, wobei die anderen Lebensmittel von demselben Lebensmitteltyp wie das Lebensmittel sind, wobei der Reifegrad des Lebensmittels bestimmt wird, ohne zerstörende Messungen des Lebensmittels vorzunehmen; und
das Übertragen des Reifegrads des Lebensmittels durch das Computersystem an einen Computer eines Anwenders zum Anzeigen auf dem Computer des Anwenders.

8. Verfahren gemäß Anspruch 7, wobei die Spektraldaten eine oder mehrere zerstörungsfreie Messungen des Lebensmittels umfassen.

9. Verfahren gemäß Anspruch 7, wobei das durch Maschinenlernen trainierte Modell eine oder mehrere Schichten umfasst, wobei jede der Schichten (i) Folgendes umfasst: Trainingsbilder der anderen Lebensmittel und (ii) Etiketten, die Lebensmittelklassifikationen für jedes der anderen Lebensmittel, die auf den Trainingsbildern abgebildet sind, anzeigen.

10. Verfahren gemäß Anspruch 7, wobei das Filtern der Spektraldaten Folgendes umfasst:
das Beschneiden der Spektraldaten;
das Versiegeln der Spektraldaten; und
das Anwenden eines Savitzky-Golay-Filters für die zweite Ableitung auf die Spektraldaten zur Rauschunterdrückung.

11. Verfahren gemäß Anspruch 7, weiterhin umfassend das Bestimmen, dass das Lebensmittel zum Verzehr geeignet ist, wenn der Reifegrad des Lebensmittels einen Schwellenwert überschreitet.

12. Verfahren gemäß Anspruch 7, weiterhin umfassend das Bestimmen, dass das Lebensmittel nicht zum Verzehr geeignet ist, wenn der Reifegrad des Lebensmittels kleiner als ein Schwellenwert ist.

13. Verfahren gemäß Anspruch 7, wobei das Verfahren umfasst:
das Empfangen des Reifegrads des Lebensmittels eines Lebensmitteltyps durch ein Computersystem, wobei der Reifegrad mit Hilfe einer zerstörungsfreien Messung des Lebensmittels und eines trainierten Modells des Lebensmitteltyps bestimmt wird, wobei das trainierte Modell mit Hilfe von einer oder mehreren zerstörenden Messungen anderer Lebensmittel des Lebensmitteltyps trainiert wurde;
das Identifizieren von Lieferketteninformationen für das Lebensmittel, die einen bereits vorhandenen Lieferkettenzeitplan und einen Bestimmungsort für das Lebensmittel umfassen, durch das Computersystem;
das Bestimmen, ob die Lieferketteninformationen für das Lebensmittel aufgrund des empfangenen Reifegrads modifiziert werden sollen, durch das Computersystem;
als Reaktion auf eine Bestimmung, dass die Lieferketteninformationen modifiziert werden sollen, das Erzeugen modifizierter Lieferketteninformationen aufgrund des empfangenen Reifegrads durch das Computersystem, wobei die modifizierten Lieferketteninformationen einen modifizierten Lieferkettenzeitplan und/oder einen modifizierten Bestimmungsort für das Lebensmittel umfassen; und
das Übertragen der modifizierten Lieferketteninformationen durch das Computersystem an einen oder mehrere Lieferkettenakteure, um die modifizierten Lieferketteninformationen zu implementieren.

14. Verfahren gemäß Anspruch 13, weiterhin umfassend:
das Bestimmen, dass das Lebensmittel zum Verzehr durch Endverbraucher geeignet ist, wenn der empfangene Reifegrad einen Schwellenwert überschreitet, durch das Computersystem; und
das Bestimmen, dass das Lebensmittel nicht zum Verzehr durch Endverbraucher geeignet ist, wenn der empfangene Reifegrad kleiner als der Schwellenwert ist, durch das Computersystem.

15. System (100) zur Bestimmung von Reifegraden bei Lebensmitteln (102) mit Hilfe von kontaktlosen Bewertungen der Lebensmittel (102), wobei das System (100) umfasst:
einen oder mehrere Penetrometer, die so konfiguriert sind, dass sie Penetrometerdaten (114) für eine Vielzahl von Testlebensmitteln (102) desselben Lebensmitteltyps messen können;
einen oder mehrere Durometer, die so konfiguriert sind, dass sie Durometerdaten (116) für die Vielzahl von Testlebensmitteln (102) desselben Lebensmitteltyps messen können;
ein oder mehrere Spektralbildgebungsgeräte (194), die so konfiguriert sind, dass sie Spektraldaten für Lebensmittel (102) desselben Lebensmitteltyps messen können; und
wenigstens ein Computersystem (190, 106), das so konfiguriert ist, dass es das Verfahren gemäß einem der Ansprüche 1 bis 12 durchführen kann.

## Revendications

1. Procédé de génération d'un modèle entraîné pour déterminer une mesure de maturité d'un aliment (102),
le procédé comprenant :
la réception (300), par un système informatique (190), (i) de données (114) de pénétromètre d'un ou plusieurs pénétromètres et (ii) de données (116) de duromètre d'un ou plusieurs duromètres pour une pluralité d'aliments de test i (102) d'un même type d'aliment, **caractérisé en ce que** le procédé comprend en outre :
la sélection (308), par le système informatique (190), de parties des données (114) de pénétromètre et des données (116) de duromètre ;
la détermination (318), par le système informatique (190), d'une mesure de maturité pour des aliments (102) du même type d'aliment sur la base des parties sélectionnées des données (114) de pénétromètre et des données (116) de duromètre ; et
la génération (320), par le système informatique (190), d'un modèle entraîné par apprentissage automatique (130) sur la base de la mesure de maturité, dans lequel le modèle entraîné par apprentissage automatique (130) corrèle des mesures destructives fournies par les parties sélectionnées des données (114) de pénétromètre et les parties sélectionnées des données (116) de duromètre avec des mesures non destructives fournies par des données spectrales pour modéliser la mesure de maturité pour les aliments (102) du même type d'aliment.

2. Procédé selon la revendication 1, dans lequel la sélection (308) de parties des données de pénétromètre et des données de duromètre comprend :
la représentation graphique des données de pénétromètre et des données de duromètre ;
l'identification d'un point d'inflexion dans les données de pénétromètre représentées graphiquement et les données de duromètre représentées graphiquement ;
la sélection de parties des données de pénétromètre et des données de duromètre sur la base du point d'inflexion ; et
le rejet de parties non sélectionnées des données de pénétromètre et des données de duromètre.

3. Procédé selon la revendication 2, dans lequel la sélection des parties des données de pénétromètre et de duromètre sur la base du point d'inflexion comprend la sélection de parties des données de pénétromètre avant le point d'inflexion et la sélection de parties des données de duromètre après le point d'inflexion.

4. Procédé selon la revendication 3, comprenant en outre le rejet de parties des données de duromètre avant le point d'inflexion et le rejet de parties des données de pénétromètre après le point d'inflexion.

5. Procédé selon la revendication 2, dans lequel la génération (320) du modèle entraîné par apprentissage automatique comprend (i) la corrélation des parties sélectionnées des données de pénétromètre avant le point d'inflexion avec une ou plusieurs longueurs d'onde de données spectrales qui correspondent à la pluralité d'aliments de test qui sont fermes et (ii) la corrélation des parties sélectionnées des données de duromètre après le point d'inflexion avec une ou plusieurs longueurs d'onde de données spectrales qui correspondent aux aliments de test qui sont mous.

6. Procédé selon la revendication 1, dans lequel le modèle entraîné par apprentissage automatique corrèle en outre les mesures destructives fournies par les parties sélectionnées des données de pénétromètre et les parties sélectionnées des données de duromètre avec au moins un élément parmi (i) un lieu d'origine, (ii) une température de stockage et (iii) des informations historiques de maturité associées aux aliments du même type d'aliment.

7. Procédé selon la revendication 1, comprenant en outre :
la réception, par un système informatique et à partir d'un dispositif d'imagerie spectrale, de données spectrales d'un aliment ;
le filtrage, par le système informatique, des données spectrales ;
la détermination, par le système informatique et sur la base de l'application du modèle entraîné par apprentissage automatique aux données spectrales filtrées, d'un niveau de maturité de l'aliment, dans lequel le modèle entraîné par apprentissage automatique a été entraîné en utilisant (i) une ou plusieurs mesures destructives d'autres aliments et (ii) des données spectrales pour les autres aliments, dans lequel les autres aliments sont du même type d'aliment que l'aliment, dans lequel le niveau de maturité de l'aliment est déterminé sans prendre de mesures destructives de l'aliment ; et
la transmission, par le système informatique à un dispositif informatique utilisateur, du niveau de maturité de l'aliment à afficher au niveau du dispositif informatique utilisateur.

8. Procédé selon la revendication 7, dans lequel les données spectrales incluent une ou plusieurs mesures non destructives de l'aliment.

9. Procédé selon la revendication 7, dans lequel
le modèle entraîné par apprentissage automatique inclut une ou plusieurs couches, dans lequel chacune des couches inclut (i) des images d'entraînement des autres aliments et (ii) des étiquettes qui indiquent des classifications d'aliments pour chacun des autres aliments représentés par les images d'entraînement.

10. Procédé selon la revendication 7, dans lequel le filtrage des données spectrales inclut :
l'ajustement des données spectrales ;
la mise à l'échelle des données spectrales ; et l'application d'un filtre de Savitzky-Golay pour la dérivée 2^{nde} aux données spectrales pour réduire le bruit.

11. Procédé selon la revendication 7, comprenant en outre la détermination que l'aliment est adapté à la consommation sur la base du niveau de maturité de l'aliment dépassant une valeur seuil.

12. Procédé selon la revendication 7, comprenant en outre la détermination que l'aliment n'est pas adapté à la consommation sur la base du fait que le niveau de maturité de l'aliment est inférieur à une valeur seuil.

13. Procédé selon la revendication 7, le procédé comprenant :
la réception, par un système informatique, du niveau de maturité de l'aliment d'un type d'aliment, dans lequel le niveau de maturité est déterminé en utilisant une mesure non destructive de l'aliment et un modèle entraîné pour le type d'aliment, dans lequel le modèle entraîné a été entraîné en utilisant une ou plusieurs mesures destructives d'autres aliments du type d'aliment ;
l'identification, par le système informatique, d'informations de chaîne d'approvisionnement pour l'aliment qui incluent un calendrier de chaîne d'approvisionnement préexistant et une destination pour l'aliment ;
la détermination, par le système informatique, s'il convient de modifier les informations de chaîne d'approvisionnement pour l'aliment sur la base du niveau de maturité reçu ;
en réponse à une détermination établissant qu'il convient de modifier les informations de chaîne d'approvisionnement, la génération, par le système informatique, d'informations de chaîne d'approvisionnement modifiées, sur la base du niveau de maturité reçu, dans lequel les informations de chaîne d'approvisionnement modifiées incluent un ou plusieurs parmi un calendrier de chaîne d'approvisionnement modifié et une destination modifiée pour l'aliment ; et
la transmission, par le système informatique, des informations de chaîne d'approvisionnement modifiées à un ou plusieurs acteurs de chaîne d'approvisionnement pour appliquer les informations de chaîne d'approvisionnement modifiées.

14. Procédé selon la revendication 13, comprenant en outre :
la détermination, par le système informatique et sur la base du fait que le niveau de maturité reçu dépasse une valeur seuil, que l'aliment est adapté à la consommation par les consommateurs finaux ; et
la détermination, par le système informatique et sur la base du fait que le niveau de maturité reçu est inférieur à la valeur seuil, que l'aliment n'est pas adapté à la consommation par les consommateurs finaux.

15. Système (100) pour déterminer les niveaux de maturité d'aliments (102) en utilisant des évaluations sans contact
des aliments (102), le système (100) comprenant :
un ou plusieurs pénétromètres configurés pour mesurer des données (114) de pénétromètre pour une pluralité d'aliments de test (102) d'un même type d'aliment ;
un ou plusieurs duromètres configurés pour mesurer des données (116) de duromètre pour la pluralité d'aliments de test (102) du même type d'aliment ;
un ou plusieurs dispositifs d'imagerie spectrale (194) configurés pour mesurer des données spectrales pour des aliments (102) du même type d'aliment ; et
au moins un système informatique (190, 106) configuré pour réaliser le procédé selon l'une quelconque des revendications 1 à 12.
